(19) Europäisches Patentamt
European Patent Office
Office européen des brevets

(11) Publication number: **0 322 582**
**A2**

(12) **EUROPEAN PATENT APPLICATION**

(21) Application number: **88119955.8**

(22) Date of filing: **30.11.88**

(51) Int. Cl.4: **C07D 409/04 , C07D 401/04 , C07D 405/04 , C07D 403/04 , C07D 417/04 , C07D 333/20 , C07D 213/36 , A61K 31/55**

Claims for the following Contracting States: ES + GR.

(30) Priority: **07.12.87 US 129820**

(43) Date of publication of application: **05.07.89 Bulletin 89/27**

(84) Designated Contracting States: **AT BE CH DE ES FR GB GR IT LI LU NL SE**

(71) Applicant: **HOECHST-ROUSSEL PHARMACEUTICALS INCORPORATED**
**Route 202-206 North**
**Somerville New Jersey 08876(US)**

(72) Inventor: **Martin, Lawrence Leo**
**R.D. 3, Box 81 Concord Road**
**Lebanon, N.J. 08833(US)**
Inventor: **Payack, Joseph Francis**
**12-D Franklin Greens**
**Somerset, N.J. 08873(US)**
Inventor: **Brucato, Salvatore**
**29 Birch Street**
**Carteret, N.J. 07008(US)**

(74) Representative: **Becker, Heinrich Karl Engelbert, Dr. et al**
**HOECHST AKTIENGESELLSCHAFT Central Patent Department P.O. Box 80 03 20**
**D-6230 Frankfurt am Main 80(DE)**

(54) **4-Heteroaryl-1,3-Benzodiazepines and 2-substituted-alpha-(heteroaryl)benzeneethanamines, a process for their preparation and their use us medicaments.**

(57) The present invention relates to 4-heteroaryl-1,3-benzodiazapines and 2-substituted-α-(heteroaryl)-benzeneethanamines and processes for the preparation thereof. The compounds have antidepressant and anticonvulsant activity and can, therefore, be used as medicaments.

EP 0 322 582 A2

# 4-HETEROARYL-1,3-BENZODIAZEPINES AND 2-SUBSTITUTED-α-(HETEROARYL)BENZENEETHANAMINES, A PROCESS FOR THEIR PREPARATION AND THEIR USE AS MEDICAMENTS

This invention relates to 4,5-dihydro-4-heteroaryl-3H-1,3-benzodiazepines. More particularly, this invention relates to 4,5-dihydro-4-heteroaryl-3H-1,3-benzodiazepines of the formula I:

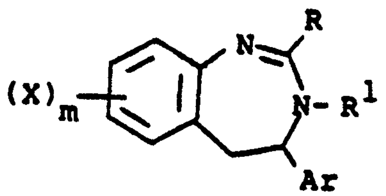

I

wherein Ar is a radical selected from the group consisting

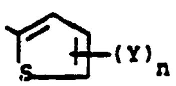 , 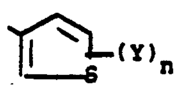 , 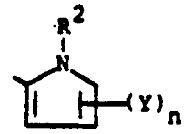 , 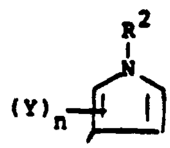

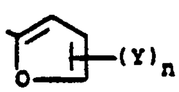 , 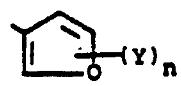 ,

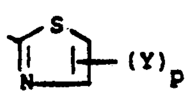 , 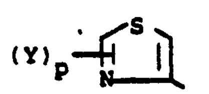 , 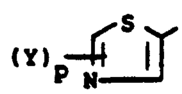 ,

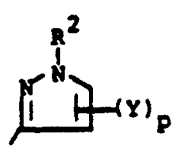 , 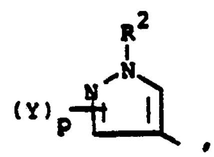 , 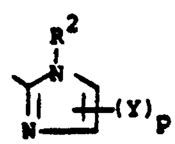 ,

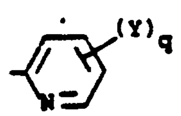 , 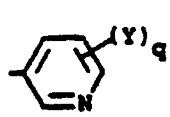 , and 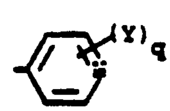

wherein $R^2$ is hydrogen, loweralkyl or loweralkanoyl, Y is selected from the group consisting of halogen, hydroxyl, loweralkyl, loweralkoxy, and trifluoromethyl, n is an integer having a value from 0 to 3 inclusive, p is an integer having a value of 0 or 1, and q is an integer having a value from 0 to 4 inclusive; X is selected from the group consisting of halogen, hydroxyl, nitro, loweralkyl, loweralkoxy, and trifluoromethyl; m is an integer having a value from 0 to 2 inclusive; R is selected from the group consisting of hydrogen, loweralkyl, aryl, aralkyl, cycloalkylloweralkyl, loweralkenyl, and loweralkynyl; and $R^1$ is selected from the group consisting of hydrogen, loweralkyl, and aralkyl, wherein for each value of m, n, p, or q each X or Y may be the same or different; the optical antipodes; geometrical isomers; or pharmaceutically acceptable acid addition salts thereof which are useful as antidepressants.

Preferred 4,5-dihydro-4-heteroaryl-3H-1,3-benzodiazepines of this invention are compounds of the formula Ia:

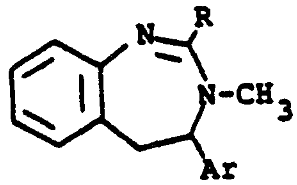

Ia

wherein Ar is selected from the group consisting of

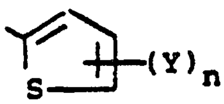 , 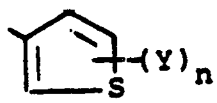 ,

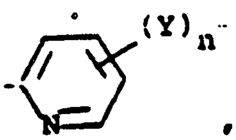 , 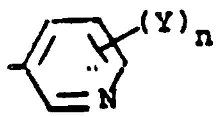 , and 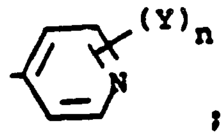 ;

wherein Y is loweralkyl, most preferably methyl, n is an integer having a value of 0 or 1, and R is selected from the group consisting of hydrogen, loweralkyl, aralkyl, and aryl; the optical antipodes; geometrical isomers; or pharmaceutically acceptable acid addition salts thereof.

Subgeneric to the 4,5-dihydro-4-heteroaryl-3H-1,3-benzodiazepines of this invention are Formula I compounds wherein

(a) Ar is

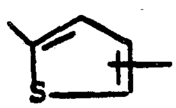 or 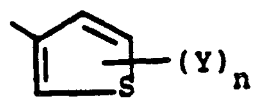

wherein Y and n are as previously described;

(b) Ar is

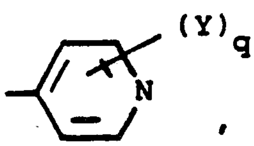 , 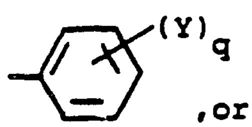 , or 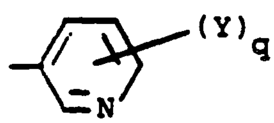

wherein Y and q are as previously described;

(c) Ar is

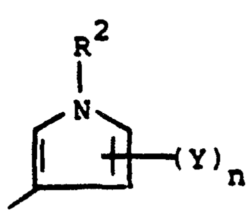 or 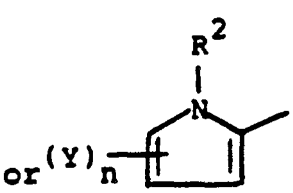

wherein $R^2$, Y and n are as previously described;

(d) Ar is

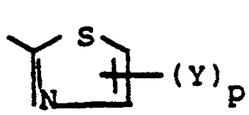 , 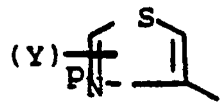 or 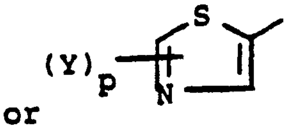

wherein Y and p are as previously described;

(e) Ar is

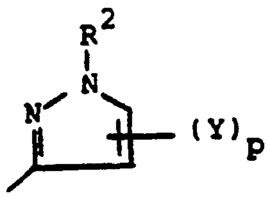 or 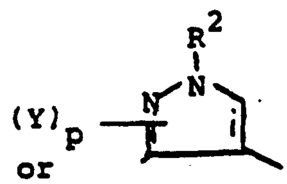

wherein $R^2$, Y and p are as previously described;

(f) Ar is

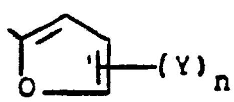 or 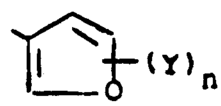

wherein Y and n are as previously described;

(g) Ar is

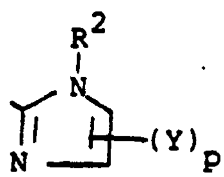

wherein $R^2$, Y and p are as previously described;

(h) R is hydrogen, loweralkyl, aryl, aralkyl, loweralkenyl or loweralkynyl;
(i) R is hydrogen, loweralkyl, aryl or aralkyl;
(j) $R^1$ is hydrogen;
(k) $R^1$ is loweralkyl, or aralkyl
(l) $R^2$ is hydrogen or loweralkyl;
(m) n is zero or 1;
(n) m is zero or 1;
(o) p is zero;
(p) q is zero or 1;
(q) X is loweralkyl;
(r) X is hydroxyl or loweralkoxy;
(s) X is halogen or trifluoromethyl;
(t) X is nitro;
(u) Y is hydroxyl or loweralkoxy;
(v) Y is loweralkyl;
(w) Y is halogen or trifluoromethyl; and
(x) Y is loweralkyl or chlorine.

In another embodiment this invention relates to compounds of the formula II

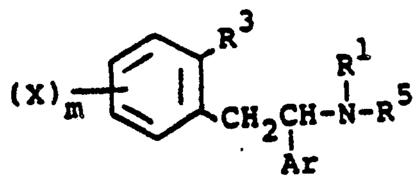

II

wherein Ar is selected from the group consisting of

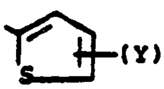 , 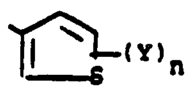 , 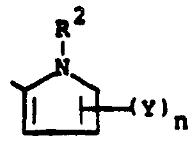 , 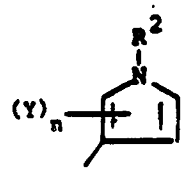

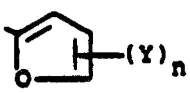 , 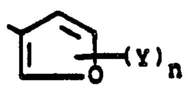 ,

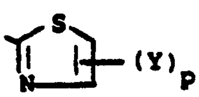 , 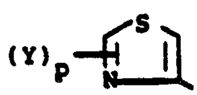 , 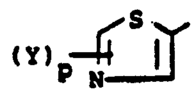 ,

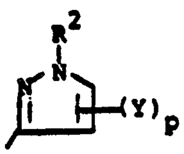 , 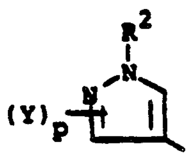 , 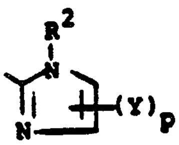 ,

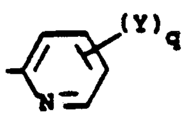 , 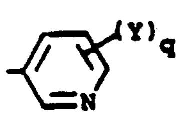 , and 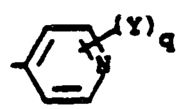

wherein $R^2$ is hydrogen, loweralkyl or loweralkanoyl, Y is selected from the group consisting of halogen, hydroxyl, loweralkyl, loweralkoxy and trifluoromethyl, n is an integer having a value from 0 to 3 inclusive, p is an integer having a value of 0 or 1, and q is an integer having a value from 0 to 4 inclusive; X is selected from the group consisting of halogen, hydroxyl, nitro, loweralkyl, loweralkoxy, and trifluoromethyl; m is an integer having a value from 0 to 3 inclusive; $R^1$ is selected from the group consisting of hydrogen, loweralkyl, and aralkyl; $R^3$ is hydroxy, loweralkoxy, amino, loweralkylamino, diloweralkylamino, or $NR^6C(O)$-$R^4$ wherein $R^4$ and $R^6$ are independently hydrogen or loweralkyl; and $R^5$ is hydrogen, loweralkyl or $C(O)R^7$ wherein $R^7$ is hydrogen or loweralkyl, wherein for each value of m, n, p or q, each X or Y may be the same or different; the geometrical isomers, optical antipodes or pharmaceutically acceptable acid addition salts thereof.

The Formula II compounds of this invention, are useful an anticonvulsants. Additionally, several of these Formula II compounds have utility as intermediates in the synthesis of the 4-heteroaryl-1,3-benzodiazepines of this invention. Subgeneric to the Formula II compounds of this invention are those compounds wherein

(aa) $R^1$ is hydrogen;
(bb) $R^1$ is loweralkyl;
(cc) $R^1$ is aralkyl;
(dd) $R^3$ is amino;
(ee) $R^3$ is loweralkylamino;

(ff) $R^3$ is diloweralkylamino;
(gg) $R^3$ is $NR^6(C(O)R^4$ wherein $R^4$ and $R^6$ are independently hydrogen or loweralkyl;
(hh) $R^3$ is hydroxy;
(ii) $R^3$ is loweralkoxy;
(jj) $R^5$ is hydrogen;
(kk) $R^5$ is $-C(O)R^7$ wherein $R^7$ is hydrogen or loweralkyl;
(ll) $R^5$ is loweralkyl;
(mm) Ar is selected from the group consisting of

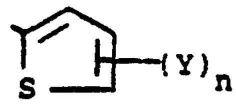 or 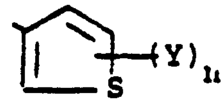

wherein n is an integer having a value of 0 or 1 and Y is loweralkyl, most preferably methyl.
(nn) Ar is

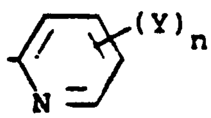, 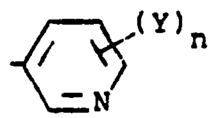 or 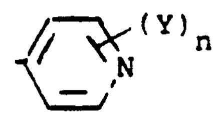

wherein n is an integer having a value of 0 or 1 and Y is loweralkyl, most preferably methyl.
(oo) Ar is

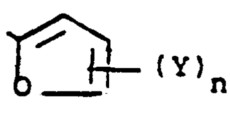 or 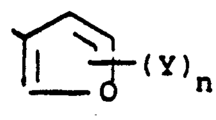

wherein n is an integer having a value of 0 or 1 and Y is loweralkyl, most preferably methyl.

A class of preferred 2-substituted-α-(heteroaryl)-benzeethanamines of this invention is represented by the formula III

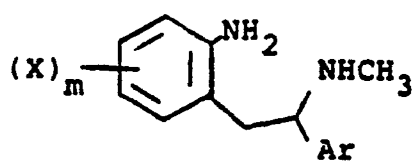

III

wherein X is halogen, preferably bromine or chlorine, m is zero or 1, and Ar is

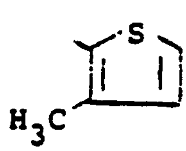 or 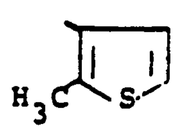

As used throughout the specification and appended claims, the term "loweralkyl" shall mean a straight or branched chain hydrocarbon group containing no unsaturation and having from 1 to 5 carbon atoms such as methyl, ethyl, 1-propyl, 2-propyl, 1-butyl, 1-pentyl, 2-pentyl.

The term "cycloalkyl" shall mean a saturated hydrocarbon group possessing at least one carbocyclic ring and having from 3 to 7 carbon atoms such as cyclopropyl, cyclobutyl, cyclopentyl, cyclohexyl, cycloheptyl.

The term "loweralkoxy" shall mean a monovalent substituent which consists of a loweralkyl group linked through an ether oxygen and having its free valence bond from the ether oxygen, such as methoxy, ethoxy, isopropoxy, t-butoxy, pentoxy. The term "aryl" shall mean phenyl or phenyl substituted with one or more chloro, bromo, fluoro, loweralkyl, methoxy, hydroxy, or trifluoromethyl groups. The term "aralkyl" refers to a radical formed by attachment of a loweralkyl function having up to 4 carbon atoms, inclusive to an aryl moiety. The term "alkanoyl" shall mean the residue of an alkyl carboxylic acid (alkanoic acid) having from 1 to 5 carbon atoms formed by the removal of the hydroxy group of the carboxylic acid moiety. Examples of alkanoyl groups include formyl, acetyl, propionyl, butyryl, pentanoyl. The term "loweralkenyl" shall mean a saturated hydrocarbon group of 1 to 5 carbon atoms having one or more carbon-carbon double bonds, and the term "alkenyl" shall mean a hydrocarbon group of 1 to 5 carbon atoms having one or more carbon-carbon triple bonds.

As shown by Reaction Scheme A, Formula II compounds wherei $R^3$ is amino, $R^5$ is hydrogen and $R^1$ is loweralkyl or aralkyl are produced by reacting a N-(2-methylphenyl)-2,2-dimethylpropanamide 1, via the corresponding dilithium adduct 2, with a heteroaryl carboxaldehyde imine 3 to produce a heteroaryl-substituted intermediate 4, hydrolysis of which yields the corresponding diamine 5.

N-(2-methylphenyl)-2,2-dimethylpropanamide 1 can be synthesized by the reaction of o-toluidine and trimethylacetyl chloride as described in U.S. Patent No. 4,374,067 to Lee et al., assigned to Hoechst-Roussel Pharmaceuticals, Inc. The reaction of primary aliphatic or aromatic amines with heteroaryl carboxaldehydes to yield heteroaryl carboxaldehyde imines is well known in the art and is analogous to the reaction of aromatic aldehydes and primary amines described, for example, in G. Hilgetag and A. Martini, Preparative Organic chemistry, John Wiley & Sons, Inc., New York, 1972, pp. 504-409.

Production of the heteroaryl-substituted intermediate 4 is conveniently accomplished by first treating N-(2-methylphenyl)-2,2-dimethylpropanamide 1 with an organolithium compound (e.g. tert-butyllithium, sec-butyllithium, n-butyllithium, and the like; n-butyllithium being preferred) and thereafter converting the resultant dilithium adduct 2 to the heteroaryl carboxaldehyde imine 3. In general, the reaction is conducted in the presence of an inert organic solvent without isolation of the intermediate dilithium adduct. Among the suitable solvents for the reaction there may be mentioned ethereal solvents, hydrocarbons, and the like, and mixtures thereof. Representative of such solvents are diethyl ether, dioxane, 1,2-dimethoxyethane, tetrahydrofuran, hexane, cyclohexane, benzene, toluene, xylene, and the like. Tetrahydrofuran, toluene, and mixtures thereof with hexane (commonly present as a solvent for the organolithium compound) are preferred. The reaction is ordinarily conducted at temperatures of from about -70 °C to about 30 °C, with temperatures of about -10 °C to about 0 °C being preferred. Typically, the amount of organolithium compound reacted is up to about 10% in excess of the 2 molar equivalents required for the reaction. Owing to the reactivity of the organolithium compound, it is recommended that the reaction be conducted under anhydrous conditions.

Hydrolysis of the heteroaryl substituted intermediate 4 to the corresponding diamine 5 is conveniently accomplished in the presence of an aqueous solvent by an appropriate mineral acid (e.g. hydrochloric acid, hydrobromic acid, sulfuric acid, 6N-hydrochloric acid being preferred) under reflux conditions. Desirably the hydrolysis is conducted under an inert atmosphere to avoid undesirable side reactions.

As further illustrated in Reaction Scheme A, Formula II compounds wherein $R^3$ is amino or -NHC(O)$R^4$, and $R^5$ is -C(O)$R^7$ can be produced by treating a diamine 5 with a suitable alkanoic acid anhydride. (e.g. formic acid anhydride, acetic acid anhydride, propionic acid anhydride, butyric acid anhydride, pentanoic acid anhydride) like). The acylation is generally conducted at temperatures of from about -30 °C to about 20 °C, preferably from about 0 °C to about 10 °C, in the presence of a suitable inert organic solvent. Suitable solvents include non-nucleophilic amines such as, for example, pyridine, lutidine, collidine, trimethyl amine. Pyridine is preferred.

Reduction of the resultant carbonyl derivatives 5a and 5b affords Formula II compounds 6a and 6b wherein $R^3$ is amino or loweralkylamino, and $R^5$ is hydrogen or loweralkyl. Reduction may be accomplished by any of several methods which are well known in the art. For example, treatment of formyl substituted derivatives 5a and 5b with a boron-tetrahydrofuran complex followed by hydrolysis with a mineral acid (e.g. concentrated hydrochloric acid).

Treatment of a formyl substituted derivative 5b with formaldehyde and succinimide followed by reduction of the resultant dioxo-pyrolidinyl substituted compound 6c, affords a derivative 6d wherein $R^3$ is methylamino. Hydrolysis of 6d with mineral acid or reduction provides compounds 6e where $R^5$ is hydrogen or loweralkyl.

Diazotization of derivatives 5b with nitrous acid and quenching into aqueous copper sulfate solution affords Formula III compounds 6f wherein $R^1$ is loweralkyl and $R^7$ is hydrogen or loweralkyl. Hydrolysis of carbonyl derivatives 6f with a mineral acid affords Formula III compounds 6g wherein $R^5$ is hydrogen.

It should be noted that, if desired, substitution of the phenyl ring of the Formula II compounds of this invention can be effected subsequent to formation of the diamine 5. For example, treatment of a phenyl-unsubstituted diamine 5 with a halosuccinimide (e.g. N-bromosuccinimide or N-chlorosuccinimide) provides the compound with one or more halide substitutes, X-.

Alternatively, as shown in Reaction Scheme B, Formula II compounds wherein $R^1$ and $R^5$ are hydrogen and $R^3$ is amino may be prepared by converting a heteroaryl-substituted 2-(2-nitrophenyl)acetophenone 7 to an oxime 8, acylating the oxime 8 with an alkanoic acid anhydride, and reducing the resultant oxime alkanoate 9 to a diamine 10.

The synthesis of heteroaryl-substituted 2-(2-nitrophenyl)acetophenones 7 is known in the art and is analogous to the synthesis of phenyl-substituted 2-(2-nitrophenyl)acetophenones described in greater detail in U.S. Patent No. 4,459,231 incorporated herein by reference.

Oxime conversion may be accomplished by any of several methods known in the art. A preferred method involves refluxing the 2-(2-nitrophenyl)acetophenone 7 in a mixture of ethyl alcohol, aqueous sodium acetate and hydroxylamine to provide the corresponding oxime 8. Alkanoic acid anhydrides suitable for acylation of the oxime 8 include formic acid anhydride, acetic acid anhydride, butyric acid anhydride, pentanoic acid anhydride, acid anhydride is preferred. The acylation is generally conducted at a temperature of from about 0°C to about 100°C, preferably from about 0°C to about 25°C, in the presence of pyridine. Reduction of the oxime alkanoate 9 may be accomplished by any of several methods known in the art, among which treatment with a boron-tetrahydrofuran complex followed by hydrolysis is preferred.

As shown in Reaction Scheme C, Formula II diamine 5 can be cyclized by reaction with a compound of the formula:

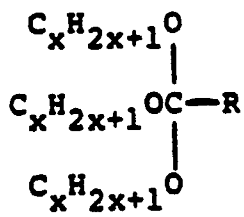

wherein R is hydrogen, loweralkyl, aralkyl or aryl, and x is 1 or 2, (e.g. trimethyl orthoformate, trimethyl orthoacetate, triethyl orthoacetate, trimethyl orthoacetate, triethyl orthoisopropionate, trimethyl orthoisobutyrate, trimethyl orthobenzoate, and the like) to provide Formula I 4,5-dihydro-4-heteroaryl-3H-1,3-benzodiazepines 12 wherein R is hydrogen, loweralkyl, aralkyl or aryl. The cyclization is generally conducted in the presence of an appropriate acid catalyst (e.g. alkanoic or alkanolic acids such as, for example, glacial acetic acid, ethanolic hydrochloric acid, methanolic hydrochloric acid, and the like, glacial acetic acid being preferred, at a temperature of from about 25°C to the reflux temperature of the reaction medium. Alternatively, the cyclization may be conducted in the presence of acetonitrile at a temperature of about 80°C in the presence of an acid catalyst.

The Formula I compounds of this invention are useful as andidepressants by virtue of their ability to elicit an antidepressant response in mammals. Antidepressant activity is demonstrated in the tetrabenazine induced ptosis assay in mice [International Journal of Neuropharmacology, 8, 73 (1969)], a standard assay for antidepressant activity.

The intraperitoneal (i.p.) dosages at which the following compounds effect a 50% inhibition from the ptosis of tetrabenazine-induced depression in mice ($ED_{50}$) are:

Table 1

| Compound | Anti-Depressant Activity $ED_{50}$ (mg/kg, i.p.) |
|---|---|
| 4,5-Dihydro-2-ethyl-3-methyl-4-(2-thienyl)-3H-1,3-benzodiazepine hydrochloride | 4.7 |
| 4,5-Dihydro-2-ethyl-3-methyl-4-(3-thienyl)-3H-1,3-benzodiazepine hydrochloride | 8.7 |
| 4,5-Dihydro-2-ethyl-3-methyl-4-(3-methyl-2-thienyl)-3H-1,3-benzodiazepine hydrochloride monohydrate | 5.3 |
| 4,5-Dihydro-2-ethyl-3-methyl-4-(4-pyridinyl-3H-1,3-benzodiazepine hemihydrate | 6.8 |
| 4,5-Dihydro-2,3-dimethyl-4-(2-methyl-5-thienyl)-3H-1,3-benzodiazepine hydrochloride | 12.2 |
| Doxepin (standard) | 3.8 |

Dosage levels which the 4-heteroaryl-1,3-benzodiazepines of this invention achieve an antidepressant response is subject to variation depending upon the particular compound employed. In general, antidepressant response may be elicited at effective oral, parenteral, or intravenous doses ranging from about 0.1 to about 50 mg/kg of body weight per day. it is to be understood, however, that for any particular subject, specific dosage regimens should be adjusted according to the individual need and the professional judgment of the person administering or supervising the administration of the aforesaid compound. It is further understood that the dosages set forth herein are exemplary only and they do not, to any extent, limit the scope or practice of the invention.

The Formula II compounds of this invention are useful as anticonvulsants due to their anticonvulsant activity in mammals. Anticonvulsant activity is measured in the male mouse using the supramaximal electroshock (SES) assay described in Arch. Int. Pharmacodyn. 92: 97-107, 1952.

Intraperitoneal doses of representative compounds of the invention and their ability to protect from the effect of SES is shown below in Table 2. $ED_{50}$ values, i.e., the doses at which, within 95% confidence intervals, 50% of the animals are protected are calculated by computerized probit analysis.

Table 2

| Activity Compound | Anticonvulsant $ED_{50}$ (mg/kg, i.p.) |
|---|---|
| 2-Amino-N-methyl-alpha-(3-methyl-2-thienyl)benzenethanamine | 4.6 |
| 2-Amino-N-methyl-alpha-(2-thienyl)benzeneethanamine dihydrochloride hemihydrate | 30.4 |
| 2-Amino-5-bromo-N-methyl-alpha-(3-methyl-2-thienyl)benzeneethanamine | 9.2 |
| Diphenylhydantoin (standard) | 3.9 |

Anticonvulsant activity is achieved when the compounds of this invention are administered to a subject requiring such treatment at an effective oral, parental or intravenous dose of from1 to 50 mg/kg of body weight per day. It is to be understood, however, that for any particular subject, specific dosage regimens should be adjusted according to the individual need and the professional judgement of the person administering or supervising the administration of the compounds of the invention. It is to be further understood that the dosages set forth herein are examples only and that they do not, to any extent, limit the scope of the practice of the invention.

The compounds of this invention, while effective themselves, may be formulated and administered in the form of their pharmaceutically acceptable addition salts for the purposes of stability, convenience or crystallization, increased solubility. Preferred pharmaceutically acceptable addition salts include salts of mineral acids, for example, hydrochloric acid, sulfuric acid, nitric acid, salts of monobasic carboxylic acids such as, for example, acetic acid, propionic acid, salts of dibasic carboxylic acids such as, for example, maleic acid, fumaric acid, and salts of tribasic carboxylic acids such as, for example, carboxysuccinic acid, citric acid.

Effective quantities of the compounds of this invention may be administered orally, for example, with an

inert diluent or with an edible carrier. They may be enclosed in gelatin capsules or compressed into tablets. For the purpose of oral therapeutic administration, the aforesaid compounds may be incorporated with excipients and used in the form of tablets, troches, capsules, elixirs, suspensions, syrups, wafers, chewing gums and the like. These preparations should contain at least 0.5% of active compound, but may be varied depending upon the particular form and may conveniently be between 4% to about 70% of the weight of the unit. The amount of active compound in such compositions is such that a suitable dosage will be obtained. Preferred compositions and preparations according to the present invention are prepared so that an oral dosage unit form contains between 1.0 and 300 milligrams of the active compound.

The tablets, pills, capsules, troches and the like may also contain the following ingredients: a binder such as microcrystalline cellulose, gum tragacanth or gelatin; an excipient such as starch or lactose, a disintegrating agent such as alginic acid, Primogel®, cornstarch, a lubricant such as magnesium stearate; a glidant such as colloidal silicon dioxide; and a sweetening agent such as sucrose or saccharin or a flavoring agent such as peppermint, methyl salicylate, or orange flavoring may be added. When the dosage unit form is a capsule, it may contain, in addition to materials of the preceeding type, a liquid carrier such as a fatty oil. Other dosage unit forms may contain other various materials which modify the physical form of the dosage unit, for example, coatings. Thus, tablets or pills may be coated with sugar, shellac, or other enteric coating agents. A syrup may contain, in addition to the active compounds, sucrose as a sweetening agent and certain preservatives, dyes and colorings and flavors. Materials used in preparing these various compositions should be pharmaceutically pure and nontoxic in the amounts used.

For the purposes of parenteral therapeutic administration, the active compounds of this invention may be incorporated into a solution or suspension. These preparations should contain at least 0.1% of active compound, but may be varied between 0.5 and about 50% of the weight thereof. The amount of active compounds in such compositions is such that a suitable dosage will be obtained. Preferred compositions and preparations according to the present invention are prepared so that a parenteral dosage unit contains between 0.5 to 100 milligrams of active compound.

The solutions or suspensions may also include the following components: a sterile diluent such as water for injection, saline solution, fixed oils, polyethylene glycols, glycerine, propylene glycol or other synthetic solvents; antibacterial agents such as benzylalcohol or methyl parabens; antioxidants such as ascorbic acid or sodium bisulfite; chelating agents such as ethylenediaminetetraacetic acid; buffers such as acetates, citrates or phosphates, and agents for the adjustment of tonicity such as sodium chloride or dextrose. The parenteral preparation can be enclosed in ampules, disposable syringes or multiple dose vials made of glass or plastic.

Compounds of the invention include:

4,5-dihydro-4-(2-thienyl)-3H-1,3-benzodiazepine;
4,5-dihydro-2-methyl-4-(3-thienyl)-3H-1,3-benzodiazepine;
4,5-dihydro-2-cyclopropylmethyl-3-methyl-4-(3-thienyl)-3H-1,3-benzodiazepine;
4,5-dihydro-3-benzyl-2-methyl-4-(2-thienyl)-3H-1,3-benzodiazepine;
4,5-dihydro-2-(4-fluorophenyl)-3-methyl-4-(4-pyridinyl)-3H-1,3-benzodiazepine;
4,5-dihydro-2,3-dimethyl-4-(4-pyrazolyl)-3H-1,3-benzodiazepine;
4,5-dihydro-2-(1-propyl)-3-methyl-4-(4-pyrazolyl)-3H-1,3-benzodiazepine;
4,5-dihydro-2-ethyl-3-methyl-4-(2-pyrrolyl)-3H-1,3-benzodiazepine;
4,5-dihydro-2,3-dimethyl-8-hydroxy-4-(3-fluoro-2-thienyl)-3H-1,3-benzodiazepine.
2-amino-N-methyl-α-(4-methyl-2-thienyl)benzeneethanamine;
2-amino-N-methyl-α-(4-methyl-3-thienyl)benzeneethanamine;
2-amino-5-fluoro-N-methyl-α-(3-methyl-2-thienyl)-benzeneethanamine;
2-amino-5-iodo-N-methyl-α-(3-methyl-2-thienyl)benzeneethanamine;
2-amino-N-methyl-5-trifluoromethyl-α-(3-methyl-2-thienyl)-benzenethanamine;
2-amino-5-methoxy-N-methyl-α-(3-methyl-2-thienyl)-benzeneethanamine;
2-amino-5-methyl-N-methyl-α-(3-methyl-2-thienyl)benzeneethanamine;
2-amino-4-fluoro-N-methyl-α-(3-methyl-2-thienyl)benzeneethanamine;
2-amino-4-chloro-N-methyl-α-(3-methyl-2-thienyl)benzeneethanamine;
2-amino-4-bromo-N-methyl-α-(3-methyl-2-thienyl)benzeneethanamine;
2-amino-4-iodo-N-methyl-α-(3-methyl-2-thienyl)benzeneethanamine;
2-amino-N-methyl-α-(3-methyl-2-thienyl)-4-trifluoromethyl-benzeneethanamine;
2-amino-4-methoxy-N-methyl-α-(3-methyl-2-thienyl)benzeneethanamine;
2-amino-4-methyl-N-methyl-α-(3-methyl-2-thienyl)benzeneethanamine;
2-amino-N-methyl-α-(1-methyl-2-pyrollyl)benzeneethanamine;
2-amino-N-methyl-α-(1-methyl-3-pyrrolyl)benzeneethanamine;

2-amino-α-(4-pyrazolyl)-N-methylbenzeneethanamine;
2-amino-N-methyl-α-(2-thiazolyl)benzeneethanamine;
2-amino-N-methyl-α-(4-thiazolyl)benzeneethanamine;
2-amino-N-methyl-α-(5-thiazolyl)benzeneethanamine;
2-amino-N-methyl-α-(2-methyl-4-thiazolyl)benzeneethanamine;
2-amino-N-methyl-α-(5-methyl-2-thiazolyl)benzeneethanamine;
2-amino-N-methyl-α-(2-methyl-5-thiazolyl)benzeneethanamine;
2-amino-α-(3-pyrazolyl)-N-methylbenzeneethanamine;
2-amino-α-(2-chloro-4-thiazolyl)-N-methylbenzeneethanamine;
2-amino-α-(5-chloro-2-thiazolyl)-N-methylbenzeneethanamine;
4-amino-α-(2-chloro-4-thiazolyl)-N-methylbenzeneethanamine;
2-amino-N-methyl-α-(4-pyrazolyl)benzeneethanamine;
2-amino-N-methyl-α-(3-pyrazolyl)benzeneethanamine;
2-amino-α-(2-imidazolyl)-N-methylbenzeneethanamine;
N-formyl-2-hydroxy-N-methyl-α-(3-methyl-2-thienyl)-benzeneethanamine;
2-hydroxy-N-methyl-α-(3-methyl-2-thienyl)benzeneethanamine;
2-methoxy-N-methyl-α-(3-methyl-2-thienyl)benzeneethanamine;
N,N-dimethyl-2-methylamino-α-(3-methyl-2-thienyl)benzeneethanamine;
4,5-dihydro-4-(2-thienyl)-3H-1,3-benzodiazepine;
4,5-dihydro-3-methyl-4-(2-thienyl)-3H-1,3-benzodiazepine;
4,5-dihydro-2-methyl-4-(2-thienyl)-3H-1,3-benzodiazepine;
4,5-dihydro-2-ethyl-7-fluoro-3-methyl-4-(3-methyl-2-thienyl)-3H-1,3-benzodiazepine;
7-chloro-4,5-dihydro-2-ethyl-3-methyl-4-(3-methyl-2-thienyl)-3H-1,3-benzodiazepine;
7-bromo-4,5-dihydro-2-ethyl-3-methyl-4-(3-methyl-2-thienyl)-3H-1,3-benzodiazepine;
4,5-dihydro-2-ethyl-3-methyl-4-(3-methyl-2-thienyl)-7-trifluoromethyl-3H-1,3-benzodiazepine;
4,5-dihydro-3,7-dimethyl-2-ethyl-4-(3-methyl-2-thienyl)-3H-1,3-benzodiazepine;
4,5-dihydro-2-ethyl-7-methoxy-4-(3-methyl-2-thienyl)-3H-1,3-benzodiazepine;
4,5-dihydro-2-ethyl-8-methoxy-4-(3-methyl-2-thienyl)-3H-1,3-benzodiazepine;
8-chloro-4,5-dihydro-2-ethyl-3-methyl-4-(3-methyl-2-thienyl)-3H-1,3-benzodiazepine;
4,5-dihydro-2-ethyl-3-methyl-4-(1-methyl-2-pyrrolyl)-3H-1,3-benzodiazepine;
4,5-dihydro-2-ethyl-3-methyl-4-(1-methyl-3-pyrrolyl)-3H-1,3-benzodiazepine;
4,5-dihydro-2-ethyl-3-methyl-4-(2-thiazolyl)-3H-1,3-benzodiazepine;
4,5-dihydro-2-ethyl-3-methyl-4-(4-thiazolyl)-3H-1,3-benzodiazepine;
4,5-dihydro-2-ethyl-3-methyl-4-(5-thiazolyl)-3H-1,3-benzodiazepine;
2-cyclopropyl-4,5-dihydro-3-methyl-4-(3-methyl-2-thienyl)-3H-1,3-benzodiazepine;
3-benzyl-4,5-dihydro-2-ethyl-4-(2-thienyl)-3H-1,3-benzodiazepine;
4,5-dihydro-2-(4-fluorophenyl)-3-methyl-4-(4-pyridinyl)-3H-1,3-benzodiazepine;
4,5-dihydro-2-ethyl-3-methyl-4-(4-pyrazolyl)-3H-1,3-benzodiazepine;
4,5-dihydro-2-ethyl-3-methyl-4-(3-pyrazolyl)-3H-1,3-benzodiazepine; and
4,5-dihydro-2-ethyl-4-(2-imidazolyl)-3-methyl-3H-1,3-benzodiazepine;

## EXAMPLES

The following examples are for illustrative purposes only and are not to be construed as limiting the invention. All percentages are by volume, unless otherwise noted.

## EXAMPLE 1

N-[2-[2-Methylamino-2-(2-thienyl)ethyl]phenyl]-2,2-dimethylpropanamide

A stirred, chilled (-10 °C) solution of 19.3 g of N-[(2-methyl)phenyl]-2,2-dimethylpropanamide in 150 ml of tetrahydrofuran was treated over 43 min with 140 ml of a 1.6M solution of n-butyllithium in hexane, while maintaining the temperature at or below 0 °C. Stirring for 2.3 hours with cooling afforded a suspension. The

stirred, cooled (-6° C) suspension was treated over 13 min with a solution of 15.02 g of N-(2-thienylmethylene)methanamine in 30 ml of toluene. The reaction mixture was stirred with cooling (1° C) for 1.5 hours, quenched by the addition of 120 ml of water and concentrated. The concentrate was extracted with dichloromethane (2 x 200 ml), and the combined extract was dried over anhydrous sodium sulfate, filtered, and evaporated to an oil. Preliminary purification of the oil was achieved by preparative high pressure liquid chromatography (hereinafter "HPLC") separations utilizing a Water's Model 500A High Pressure Liquid Chromatograph (silica gel; eluting with 10% methanol in ethyl acetate). The appropriate fractions from each separation were combined and concentrated to an oil. The purification procedure was repeated utilizing 5% methanol in ethyl acetate as the eluent to yield 11.71 g of N-[2-[2-methylamino-2-(2-thienyl)ethyl]phenyl]-2,2-dimethylpropanamide as a solid, mp 78.5-82° C.

| ANALYSIS: | | | |
|---|---|---|---|
| Calculated for $C_{18}H_{24}N_2OS$: | 68.32%C | 7.64%H | 8.85%N |
| Found: | 67.96%C | 7.69%H | 8.74%N |

## EXAMPLE 2

2-Amino-N-methyl-α-(2-thienyl)benzeneethanamine dihydrochloride hemihydrate

A stirred suspension of 11.28 g of N-[2-[2-methylamino-2-(2-thienyl-ethyl]phenyl]-2,2-dimethylpropanamide in 100 ml of 6N hydrochloric acid was refluxed under nitrogen for 8 hours. The mixture was cooled, decanted over crushed ice and water (200 ml), basified by the addition of 50% sodium hydroxide solution, and extracted with dichloromethane (3 x 150 ml). The combined extract was dried over anhydrous sodium sulfate, filtered, and concentrated to an oil. The oil was purified by means of HPLC on a Water's Model 500A High Pressure Liquid Chromatograph (silica gel) utilizing methanol as the eluent. Concentration of the appropriate fractions yielded 5.96 g of 2-amino-N-methyl-α-(2-thienyl)benzeneethanamine. A sample of the product (2 g) in methanol (5 ml) was treated with ethereal hydrogen chloride and a solid was precipitated by further dilution with anhydrous ether. Recrystallization from absolute ethanol gave 2.12 g of 2-amino-N-methyl-α-(2-thienyl)benzeneethanamine dihydrochloride hemihydrate, mp 210-215° C (dec.).

| ANALYSIS: | | | |
|---|---|---|---|
| Calculated for $C_{13}H_{16}N_2S \cdot 2HCl \cdot 0.5H_2O$: | 49.68%C | 6.09%H | 8.91%N |
| Found: | 49.32%C | 5.94%H | 8.87%N |

## EXAMPLE 3

4,5-Dihydro-2,3-dimethyl-4-(2-thienyl)-3H-1,3-benzodiazepine

A stirred solution of 3.06 g of 2-amino-N-methyl-α-(2-thienyl)benzeneethanamine (prepared as described in Example 2) and 12.72 g of triethyl orthoacetate was treated rapidly with 5 ml of glacial acetic acid. The resulting solution was refluxed for 8 hours and allowed to stand at room temperature overnight. The solution was concentrated on a rotary evaporator and the residual syrup was dissolved in 50 ml of 10% hydrochloric acid. The solution was washed with diethyl ether (2 x 30 ml), basified with 10% sodium hydroxide solution, and extracted with dichloromethane (2 x 50 ml). The combined organic phase was dried over anhydrous sodium sulfate, filtered, and concentrated to an oil which crystallized under refrigeration. The crystalline product was purified by HPLC (Water's Associates Prep LC/System 500A; silica gel; sample applied in dichloromethane; 2% triethylamine in methanol as the eluent). Concentration of the appropriate

fractions yielded an oil which crystallized on trituration with hexane. The compound was isolated, washed with hexane, and dried in vacuo at room temperature to afford 2.05 g of 4,5-dihydro-2,3-dimethyl-4-(2-thienyl)-3H-1,3-benzodiazepine, mp 106.5-108.5° C.

| ANALYSIS: | | | |
|---|---|---|---|
| Calculated for $C_{15}H_{16}N_2S$: | 70.28%C | 6.29%H | 10.93%N |
| Found: | 70.61%C | 6.30%H | 10.91%N |

EXAMPLE 4

4,5 Dihydro-3-methyl-2-(1-methylethyl-4-(2-thienyl)-3H-1,3-benzodiazepine hydrochloride

A stirred solution of 3.45 g of 2-amino-N-methyl-α-(2-thienyl)benzeneethaneamine and 13.34g of trimethyl orthoisobutyrate was treated rapidly with 3.8 ml of glacial acetic acid. The reaction mixture was refluxed for 8 hours and cooled to room temperature overnight. The mixture was concentrated on a rotary evaporator at 70° C. After standing overnight under refrigeration, the residue was dissolved in 60 ml of 10% hydrochloric acid solution and extracted with diethyl ether (2 x 50 ml). The aqueous phase was basified with 10% sodium hydroxide solution and extracted with dichloromethane (2 x 75 ml). The combined organic phase was dried over anhydrous sodium sulfate, filtered, and concentrated to an oil. The oil was purified by HPLC (Water's Associates Prep LC/System 500; silica gel; sample applied in dichloromethane; methanol as eluent). Concentration of the appropriate fractions yielded 1.08 g of 4,5-dihydro-3-methyl-2-(1-methylethyl)-4-(2-thienyl)-3H-1,3-benzodiazepine as an oil.

The oil was dissolved in methanol (2 ml) and the solution was treated with a slight excess of ethereal hydrogen chloride, followed by dilution with 40 ml of anhydrous diethyl ether. Upon dilution, separate oil and ether phases formed. The oil phase was separated and triturated with three successive portions of diethyl ether to afford a semisolid which was recrystallized from propionitrile to yield 0.54 g of crystalline solid. Work-up of propionitrile mother liquor afforded an additional 0.24 g of crystals. The final propionitrile mother liquor and combined ethereal phases were concentrated to an oil which was recrystallized from propionitrile to yield an additional 0.11 g of crystals. The three crops of crystals were mixed together to yield 0.89 of 4.5-dihydro-3-methyl-2-(1-methylethyl)-4-(2-thienyl)-3H-1,3-benzodiazepine hydrochloride, mp 202-205.5° C.

| ANALYSIS: | | | |
|---|---|---|---|
| Calculated for $C_{17}H_{20}N_2S \cdot HCl$: | 63.63%C | 6.60%H | 8.73%N |
| Found: | 63.43%C | 6.62%H | 8.72%N |

EXAMPLE 5

4,5-Dihydro-3-methyl-2-(1-propyl)-4-(2-thienyl)-3H-1,3-benzodiazepine hydrochloride

A stirred solution of 3.36 g of 2-amino-N-methyl-α-(2-thienyl)benzeneethanamine (prepared as described in Example 2) and 12.89 g of trimethyl orthobutyrate was treated rapidly with 3.5 ml of glacial acetic acid. The resulting solution was refluxed for 8 hours, cooled, and concentrated on a rotary evaporator at 70° C. The residue was partitioned between 10% hydrochloric acid solution and diethyl ether. After extraction of the aqueous phase with a second portion of diethyl ether, the solution was basified with 10% sodium hydroxide solution and extracted with dichloromethane (2 x 100 ml).

The combined organic phase was dried over anhydrous sodium sulfate, filtered, and concentrated to an

oil. The oil was purified by HPLC (Water's Associates Prep LC/System 500; silica gel; and eluted with methanol followed by 2% triethylamine in methanol). Concentration of the appropriate fractions yielded 2.85 g of 4,5-dihydro-3-methyl-2-(1-propyl)-4-(2-thienyl)-3H-1,3-benzodiazepine as an oil.

The oil was dissolved in diethyl ether and treated with ethereal hydrogen chloride. The resulting precipitate was triturated with diethyl ether until solidification was complete, isolated by vacuum filtration, and dried in vacuo at 40° C. Recrystallization from acetonitrile yielded 2.21 g of 4,5-dihydro-3-methyl-2-(1-propyl)-4-(2-thienyl)-3H-1,3-benzodiazepine hydrochloride, mp 222-223.5° C.

| ANALYSIS: | | | |
|---|---|---|---|
| Calculated for $C_{17}H_{20}N_2S.HCl$: | 63.63%C | 6.60%H | 8.73%N |
| Found: | 63.49%C | 6.60%H | 8.75%N |

## EXAMPLE 6

N-[2-[2-(Methylamino)-2-(3-thienyl)ethyl]phenyl]-2,2-dimethylpropanamide

To a chilled, solution (-50° C) of 19.13 g of 2,2-dimethyl-N-[(2-methyl)phenyl]propanamide in 200 ml of tetrahydrofuran was added, dropwise over 1.5 hours, 88 ml of a 2.5 M solution of n-butyllithium in hexane. Upon completion of the addition, the resultant solution was stirred for 2 hours with cooling. A solution of 15.0 g of 3-thiophene-carboxaldehyde methylimine in 30 ml of toluene, was then added, dropwise over 15 minutes, to the cooled solution. Upon completion of the 3-thiophene-carboxaldehyde methylimine addition, the reaction mixture was stirred for 20 minutes at 0° C, quenched by the addition of 300 ml of water, concentrated, and extracted with dichloromethane. The combined extract was dried over anhydrous magnesium sulfate, filtered, and concentrated to an oil. HPLC of the oil (Water's Associates Prep LC/System 500; silica gel; methanol as the eluent) afforded a semipurified product which was subsequently chromatographed under otherwise identical conditions utilizing ethyl acetate, followed by methanol as eluents. Concentration of the appropriate fractions and trituration with hexane yielded 18.0 g of N-[2-[2-(methylamino)-2-(3-thienyl)ethyl]-phenyl]-2,2-dimethylpropanamide, mp 81-84° C.

| ANALYSIS: | | | |
|---|---|---|---|
| Calculated for $C_{18}H_{24}N_2OS$: | 68.32%C | 7.64%H | 8.85%N |
| Found: | 68.25%C | 7.75%H | 8.71%N |

## EXAMPLE 7

2-Amino-N-methyl-α-(3-thienyl)benzeneethanamine

A solution of 11.0 g of N-[2-[2-(methylamino)-2-(3-thienyl)ethyl]phenyl-2,2-dimethylpropanamide in 150 ml of 6N hydrochloric acid was refluxed for 8 hours. The solution was then decanted into an ice-water mixture (500 ml) and basified with 50% sodium hydroxide solution. The aqueous mixture was extracted with dichloromethane, dried over anhydrous magnesium sulfate and concentrated to an oil.

Purification was accomplished by preparative HPLC (Water's Associates Prep LC/System 500, silica gel, methanol eluent). Concentration of the appropriate fractions yielded 3.21 g of 2-amino-N-methyl-α-(3-thienyl)benzeneethanamine as an oil.

| ANALYSIS: | | | |
|---|---|---|---|
| Calculated for $C_{13}H_{16}N_2S$: | 67.20%C | 6.94%H | 12.06%N |
| Found: | 67.17%C | 6.93%H | 11.84%N |

EXAMPLE 8

4,5-Dihydro-2,3-dimethyl-4-(3-thienyl)-3H-1,3-benzodiazepine

A solution of 6.50 of 2-amino-N-methyl-α-(3-thienyl)benzeethanamine and 15.5 ml of triethyl orthoacetate was treated with glacial acetic acid (9 ml). After refluxing for eight hours, the volatile components were removed at 70 °C on a rotary evaporator. The residual oil was decanted into an ice-water mixture and basified with 10% sodium hydroxide solution. The aqueous mixture was extracted with dichloromethane and the organic phase was dried over anhydrous magnesium sulfate and concentrated to an oil.

Purification was accomplished by preparative HPLC (Water's Associates Prep LC/System 500, silica gel, elution with 2% triethylamine in methanol). Concentration of the appropriate fractions afforded a solid which was triturated with hexane and dried in vacuo at 50 °C to yield 3.79 g of 4,5-dihydro-2,3-dimethyl-4-(3-thienyl)-3H-1,3-benzodiazepine, mp 113-117 °C.

| ANALYSIS: | | | |
|---|---|---|---|
| Calculated for $C_{15}H_{16}N_2S$: | 70.27%C | 6.29%H | 10.93%N |
| Found: | 69.88%C | 6.30%H | 10.82%N |

EXAMPLE 9

4,5-Dihydro-2-ethyl-3-methyl-4-(2-thienyl)-3H-1,3-benzodiazepine hydrochloride

A stirred solution of 2.47 g of 2-amino-N-methyl-α-(2-thienyl)benzeneethanamine and 11.21 g of triethyl orthopropionate was treated rapidly with glacial acetic acid (4 ml). The solution was refluxed for 5 hours, allowed to stand overnight at room temperature, and concentrated on a rotary evaporator. A solution of the residual oil and 10% hydrochloric acid (50 ml) was extracted with ether (2 x 50 ml), basified with 10% sodium hydroxide solution and extracted with dichloromethane (2 x 50 ml). The combined organic phase was dried over anhydrous sodium sulfate, filtered, and concentrated to an oil.

Purification was accomplished by preparative HPLC (Water's Associates Prep LC/System 500, silica gel, sample applied in dichloromethane, and eluted with 2% triethylamine in methanol). Concentration of the appropriate fractions yielded 4,5-dihydro-2-ethyl-3-methyl-4-(2-thienyl)-3H-1,3-benzodiazepine as an oil. The oil was solubilized in 30 ml of anhydrous diethyl ether and treated with excess ethereal hydrogen chloride to precipitate the corresponding hydrochloride salt. The precipitate was washed with ether, isolated by vacuum filtration, washed again with ether and dried to vacuo at 40 °C over sodium hydroxide pellets to give 1.86 g of 4,5-dihydro-2-ethyl-3-methyl-4-(2-thienyl)-3H-1,3-benzodiazepine hydrochloride, mp 216-219 °C.

| ANALYSIS: | | | |
|---|---|---|---|
| Calculated for $C_{16}H_{18}N_2S \cdot HCl$: | 62.63%C | 6.24%H | 9.13%N |
| Found: | 62.24%C | 6.24%H | 9.02%N |

EXAMPLE 10

4,5-Dihydro-3-methyl-2-(1-propyl)-4-(3-thienyl)-3H-1,3-benzodiazepine

A solution of 4.80 g of 2-amino-N-methyl-α-(3-thienyl)benzeneethanamine and 18.35 ml of trimethyl orthobutyrate was treated with 6.90 ml of glacial acetic acid. After refluxing for 8 hours, the volatile components were removed on a rotary evaporator. The residual oil was acidified with 10% hydrochloric acid solution, and extracted with diethyl ether (2 x 100 ml). The aqueous layer was basified with 10% sodium hydroxide solution, and extracted with dichloromethane (3 x 100 ml). The organic phase was dried over anhydrous sodium sulfate, filtered, and evaporated to an oil.

Purification was accomplished by preparative HPLC (Water's Associates Prep LC/System 500A, silica gel, elution with 2% triethylamine in methanol). Concentration of the appropriate fractions yielded an oil which solidified under refrigeration. Trituration of the solid with hexane afforded granular crystals which were collected, washed with hexanes, and dried in vacuo to yield 4.08 g of 4,5-dihydro-3-methyl-2-(1-propyl)-4-(3-thienyl)-3H-1,3-benzodiazepine, mp 44.5-46.5° C.

| ANALYSIS: | | | |
|---|---|---|---|
| Calculated for $C_{17}H_{20}N_2S$: | 71.78%C | 7.09%H | 9.85%N |
| Found: | 71.65%C | 6.94%H | 9.92%N |

EXAMPLE 11

4,5-Dihydro-3-methyl-2-(1-methylethyl)-4-(3-thienyl)-3H-1,3-benzodiazepine hydrochloride monohydrate

A solution of 4.78 g of 2-amino-N-methyl-α-(3-thienyl) benzeneethanamine and 16.77 g of trimethyl orthoisobutyrate was treated with glacial acetic acid (6.87 ml). After refluxing for 8 hours, the volatile components were removed on a rotary evaporator. The residual oil was acidified with 10% hydrochloric acid aqueous solution and extracted with diethyl ether (2 x 100 ml). The aqueous solution was basified with 10% sodium hydroxide aqueous soluton to a pH of 12, and extracted with dichloromethane (3 x 100 ml). The combined dichloromethane layers were washed with 100 ml of water, dried over anhydrous sodium sulfate, and concentrated to an oil.

Purification was accomplished by preparative HPLC (Water's Associates Prep LC/System 500A; silica gel; elution with 2% triethylamine/methanol). Concentration of the appropriate fractions yielded 4,5-dihydro-3-methyl-2-(1-methylethyl)-4-(3-thienyl)-3H-1,3-benzodiazepine. The oil was converted to the corresponding hydrochloride salt which was triturated with ether to yield 2.90 g of 4,5-dihydro-3-methyl-2-(1-methylethyl)-4-(2-thienyl)-3H- 1,3-benzodiazepine hydrochloride monohydrate, mp 119-122° C.

| ANALYSIS: | | | |
|---|---|---|---|
| Calculated for $C_{17}H_{20}N_2S \cdot HCl \cdot H_2O$: | 60.24%C | 6.84%H | 8.27%N |
| Found: | 60.20%C | 6.59%H | 8.31%N |

EXAMPLE 12

4,5-Dihydro-3-methyl-2-phenyl-4-(3-thienyl)-3H-1,3-benzodiazepine hydrochloride

A solution of 6.50 g of 2-amino-N-methyl-α-(3-thienyl)benzeethanamine and 14.5 ml of trimethyl orthobenzoate was treated with glacial acetic acid (9 ml). After refluxing for eight hours, the volatile components were removed at 70 °C on a rotary evaporator. The residual oil was decanted into an ice-water mixture and basified with 10% sodium hydroxide solution. The aqueous mixture was extracted with dichloromethane and the organic phase was dried over anhydrous magnesium sulfate, filtered, and concentrated to an oil.

Purification was accomplished by preparative HPLC (Water's Associates Prep LC/System 500A, silica gel, eluted with methanol). Concentration of the appropriate fractions yielded 4,5-dihydro-3-methyl-2-phenyl-4-(3-thienyl)-3H-1,3-benzodiazepine as an oil. The oil was dissolved in methanol and treated dropwise with ethereal hydrogen chloride to precipitate the corresponding hydrochloride salt. The salt was collected by vacuum filtration, washed with ether, and dried overnight under vacuum to yield 4.49 g of 4,5-dihydro-3-methyl-2-phenyl-4-(3-thienyl)-3H-1,3-benzodiazepine hydrochloride, mp 266-269 °C.

| ANALYSIS: | | | |
|---|---|---|---|
| Calculated for $C_{20}H_{18}N_2S \cdot HCl$: | 67.68%C | 5.40%H | 7.90%H |
| Found: | 67.26%C | 5.46%H | 7.78%N |

EXAMPLE 13

4,5-Dihydro-2-ethyl-3-methyl-4-(3-thienyl)-3H-1,3-benzodiaze pine hydrochloride

A solution of 6.00 g of 2-amino-N-methyl-α-(3-thienyl)benzeneethanamine and 10 ml of triethyl orthopropionate, was treated with 8 ml of glacial acetic acid. After refluxing for eight hours, the volatile components were removed at 70 °C on a rotary evaporator. The residual oil was decanted into an ice-water mixture and basified with 10% sodium hydroxide solution. The aqueous mixture was extracted with dichloromethane and the organic phase was dried over anhydrous magnesium sulfate, filtered, and concentrated to provide 4,5-dihydro-2-ethyl-3-methyl-4-(3-thienyl)-3H-1,3-benzodiazepine as an oil.

Purification was accomplished by preparative HPLC (Water's Associates Prep LC/System 500A, silica gel, utilizing with 2% triethylamine in methanol as the eluent). Concentration of the appropriate fractions yielded an oil which was dissolved in methanol and treated dropwise with ethereal hydrogen chloride. The resultant precipitate was washed with ether and dried in vacuo to yield 3.89 of 4,5-dihydro-2-ethyl-3-methyl-4-(3-thienyl)-3H-1,3-benzodiazepine hydrochloride, mp 242-244 °C.

| ANALYSIS: | | | |
|---|---|---|---|
| Calculated for $C_{16}H_{18}N_2S.HCl$: | 62.63%C | 6.24%H | 9.13%N |
| Found: | 62.71%C | 6.26%H | 9.06%N |

EXAMPLE 14

N-[2-[2-(Methylamino)-2-(3-methyl-2-thienyl)ethyl]phenyl]-2,2-dimethylpropanamide

A chilled solution (-6 °C) of 76.0 g of 2,2-dimethyl-N-[(2-methyl)phenyl]propanamide in 600 ml of tetrahydrofuran was treated dropwise over 1.25 hours with 344 ml of a 2.5 M solution of n-butyllithium in hexanes. After the addition was complete, the resultant solution was stirred for 2 hours with cooling and then was treated dropwise over 20 minutes, with a solution of 6.0 g of 3-methyl-2-thiophenecarboxaldehyde methylimine in 30 ml of toluene. After the addition was complete, the mixture was stirred for an additional 20 minutes. The reaction was quenched by the rapid addition of water. After concentration to remove the

organic solvents, the residual oil-water mixture was extracted with dichloromethane. The combined organic phase was dried over anhydrous magnesium sulfate, filtered, and concentrated to an oil.

Purification was accomplished by preparative HPLC (Water's Associates Prep LC/System 500, silica gel, eluted with ethyl acetate). Concentration of the appropriate fractions afforded an oil which was further purified by preparative HPLC to yield 70 g of N-[2-[2-(methylamino)-2-(3-methyl-2-thienyl)ethyl]phenyl]-2,2-dimethylpropanamide as an oil.

| ANALYSIS: | | | |
|---|---|---|---|
| Calculated for $C_{19}H_{26}N_2OS$: | 69.05%C | 7.93%H | 8.48%N |
| Found: | 68.80%C | 7.80%H | 8.13%N |

EXAMPLE 15

2-Amino-N-methyl-α-(3-methyl-2-thienyl)benzeneethanamine

A solution of 35.0 g of N-[2-[2-(methylamino)-2-(3-methyl-2-thienyl)ethyl]phenyl]-2,2-dimethylpropanamide in 600 ml of 6N hydrochloric acid was refluxed for 8 hours. The solution was then decanted into an ice-water mixture and basified with 50% sodium hydroxide solution. The aqueous mixture was extracted with dichloromethane (2 L), dried over anhydrous magnesium sulfate and concentrated to an oil.

Purification was accomplished by preparative HPLC (Water's Associates Prep LC/System 500A, silica gel, eluted with methanol). Concentration of the appropriate fractions yielded 6.42 g of 2-amino-N-methyl-α-(3-methyl-2-thienyl)-benzeneethanamine as an oil.

| ANALYSIS: | | | |
|---|---|---|---|
| Calculated for $C_{14}H_{18}N_2S$: | 68.25%C | 7.36%H | 11.37%N |
| Found: | 68.35%C | 7.26%H | 11.35%N |

EXAMPLE 16

4,5-Dihydro-2,3-dimethyl-4-(3-methyl-2-thienyl)-3H-1,3-benzodiazepine hydrochloride monohydrate

A solution of 4.42 g of 2-amino-N-methyl-α-(3-methyl-2-thienyl)benzeneethanamine and 10 ml of triethyl orthoacetate was treated with glacial acetic acid (8 ml). After refluxing for eight hours, the volatile components were removed at 70°C on a rotary evaporator. The residual oil was decanted into an ice-water mixture and basified with 10% sodium hydroxide solution. The aqueous mixture was extracted with dichloromethane, dried over anhydrous magnesium sulfate and concentrated to an oil.

Purification was accomplished by preparative HPLC (Water's Associates Prep LC/System 500, silica gel, eluted with 2% triethylamine in methanol). Concentration of the appropriate fractions yielded 4,5-dihydro-2,3-dimethyl-4-(3-methyl-2-thienyl)-3H-1,3-benzodiazepine as an oil. The corresponding hydrochloride salt was made by dissolving the oil in methanol and treating with ethereal hydrogen chloride. After diluting with anhydrous ether, a precipitate separated. The solid was collected and dried overnight at 40°C under vacuum to yield 2.52 g of 4,5-dihydro-2,3-dimethyl-4-(3-methyl-2-thienyl)-3H-1,3-benzodiazepine hydrochloride monohydrate, mp 140-142°C.

| ANALYSIS: | | | |
|---|---|---|---|
| Calculated for $C_{16}H_{18}N_2S \cdot HCl \cdot H_2O$: | 59.14%C | 6.51%H | 8.62%N |
| Found: | 59.36%C | 6.25%H | 8.56%N |

EXAMPLE 17

4,5-Dihydro-2-ethyl-3-methyl-4-(3-methyl-2-thienyl)-3H-1,3-benzodiazepine hydrochloride monohydrate

A stirred solution of 2.94 g of 2-amino-N-methyl-α-(3-methyl-2-thienyl)benzeneethanamine and 12.58 g of triethyl orthopropionate was stirred rapidly with glacial acetic acid (4.0 ml). The solution was refluxed for 8 hours, cooled to room temperature and concentrated on a rotary evaporator at 80°C. The residue was slurried with ether and treated with 50 ml of 10% hyrochloric acid solution. The aqueous phase was washed with ether, basified with 10% sodium hydroxide solution (60 ml), and extracted with dichloromethane (2 x 50 ml). The combined organic phase was dried over anhydrous sodium sulfate, filtered and concentrated to an oil.

Purification was accomplished by preparative HPLC (Water's Associates Prep LC/System 500A; silica gel; sample applied in dichloromethane (20 ml); elution with 2% triethylamine in methanol. Concentration of the appropriate fractions yielded 4,5-dihydro-2-ethyl-3-methyl-4-(3-methyl-2-thienyl)-3H-1,3-benzodiazepine as an oil. The oil was dissolved in methanol (5 ml) and ether (2 ml), and the resulting solution treated with a slight excess of ethereal hydrogen chloride. Further dilution with ether precipitated the corresponding hydrochloride salt. The precipitate was isolated by vacuum filtration, washed twice with ether, and dried in vacuo at 40°C to yield 1.9 g of 4,5-dihydro-2-ethyl-3-methyl-4-(3-methyl-2-thienyl)-3H-1,3-benzodiazepine hydrochloride monohydrate, mp 185-206°C.

| ANALYSIS: | | | |
|---|---|---|---|
| Calculated for $C_{17}H_{20}N_2S \cdot HCl \cdot H_2O$: | 60.25%C | 6.84%H | 8.27%N |
| Found: | 60.15%C | 6.88%H | 8.46%N |

EXAMPLE 18

4,5-Dihydro-3-methyl-2-(1-methylethyl)-4-(3-methyl-2-thienyl)-3H-1,3-benzodiazepine hydrochloride

A solution of 5.86 g of 2-amino-N-methyl-α-(3-methyl-2-thienyl)benzeneethanamine and 19.56 g of trimethyl orthoisobutyrate was treated rapidly with 7.94 ml of glacial acetic acid. The solution was refluxed for 8 hours under nitrogen and concentrated on a rotary evaporator at 40°C. The residual oil was acidified with 10% hydrochloric acid solution and extracted with ether (3 x 100 ml). The aqueous phase was basified with 10% sodium hydroxide solution and extracted with dichloromethane (3 x 100 ml). The organic phases was dried over anhydrous sodium sulfate, filtered and concentrated to an oil. Purification was accomplished by HPLC (Water's Associates Prep LC/System 500A, silica gel, utilizing methanol followed by 2% triethylamine in methanol as successive eluents). Concentration of the appropriate fractions yielded 1.88 g of 4,5-dihydro-3-methyl-2-(1-methylethyl)-4-(3-methyl-2-thienyl)-3H-1,3-benzodiazepine as an oil. The oil was treated with ethereal hydrogen chloride and the resulting precipitate was triturated with ether to yield 1.80 g of 4,5-dihydro-3-methyl-2-(1-methylethyl)-4-(3-methyl-2-thienyl)-3H-1,3-benzodiazepine hydrochloride, mp 240.5-241.0°C.

| ANALYSIS: | | | |
|---|---|---|---|
| Calculated for $C_{18}H_{22}N_2S.HCl$: | 64.55%C | 6.92%H | 8.37%N |
| Found: | 64.28%C | 6.96%H | 8.27%N |

## EXAMPLE 19

4,5-Dihydro-3-methyl-4-(3-methyl-2-thienyl)-2-phenyl-3H-1,3-benzodiazepine hydrochloride

A stirred solution of 2.19 g of 2-amino-N-methyl-α-(3-methyl-2-thienyl)benzeneethanamine and 9.88 g of trimethyl orthobenzoate was treated rapidly with 2.4 ml of glacial acetic acid, and heated under reflux for 8 hours. The solution was concentrated at 75° C on a rotary evaporator (vacuum pump) to a syrup. The syrup was then partioned between 10% hydrochloric acid solution (50 ml) and ether. The aqueous phase was extracted again with ether, basified with 10% sodium hydroxide solution (60 ml) and extracted with dichloromethane (2 x 100 ml). The combined organic phase was dried over anhydrous sodium sulfate, filtered, and concentrated to an oil. The oil was purified by preparative HPLC (Water's Associates Prep LC/System 500A, silica gel, samrple applied in dichloromethane, 2% triethylamine in methanol as the eluent). Concentration of the appropriate fractions yielded 2.70 g of 4,5-dihydro-3-methyl-4-(3-methyl-2-thienyl)-2-phenyl-3H-1,3-benzodiazepine as an oil.

A solution of the oil and methanol (5 ml) was treated with a slight excess of ethereal hydrogen chloride. Further dilution with ether precipitated the corresponding hydrochloride salt. The precipitate was isolated and dried in vacuo at 40° C to yield 2.13 g of 4,5-dihydro-3-methyl-4-(3-methyl-2-thienyl)-2-phenyl-3H-1,3-benzodiazepine hydrochloride, mp 221-224° C.

| ANALYSIS: | | | |
|---|---|---|---|
| Calculated for $C_{21}H_{20}N_2S.HCl$: | 68.37%C | 5.74%H | 7.59%N |
| Found: | 67.95%C | 6.01%H | 7.57%N |

## EXAMPLE 20

N-[2-[2-(Methylamino)-2-(2-pyridinyl)ethyl]phenyl]-2,2-dimethylpropanamide

A chilled solution (-5° C) of 38.26 g of 2,2-dimethyl-N-[(2-methyl)phenyl]propanamide in 300 ml of tetrahydrofuran was treated dropwise over 1.25 hour with 176 ml of a 2.5 M solution of n-butyllithium in hexanes. After the addition was complete, the resultant solution was stirred for 2.5 hours with cooling and then was treated dropwise over 15 minutes with a solution of 28.84 g of 2-pyridinecarboxaldehyde methylimine in 60 ml of toluene. The solution was stirred for an additional 15 minutes, quenched by the rapid addition of water (250 ml), and concentrated. The residue was extracted with dichloromethane (750 ml). The organic phase was dried over anhydrous magnesium sulfate, and concentrated to an oil.

Purification was accomplished by preparative HPLC (Water's Associates Prep LC/System 500, silica gel, elution with methanol). Concentration of the appropriate fractions yielded an oil which was further purified by preparative HPLC until free of impurities to yield 10 g of N-[2-[2-(methylamino)-2-(2-pyridinyl)-ethyl]phenyl]-2,2-dimethylpropanamide as an oil.

| ANALYSIS: | | |
|---|---|---|
| Calculated for $C_{19}H_{25}N_3O$: | 73.28%C | 8.09%H |
| Found: | 73.84%C | 7.89%H |

EXAMPLE 21

2-Amino-N-methyl-α-(2-pyridinyl)benzeneethanamine

A solution of 8.0 g of N-[2-[2-(methylamino)-2-(2-pyridinyl)ethyl]phenyl]-2,2-dimethylpropanamide in 100 ml of 6N hydrochloric acid was refluxed for 8 hours. The solution was then decanted into ice-water (250 ml) and basified with 50% sodium hydroxide solution. The aqueous mixture was extracted with dichloromethane (500 ml), dried over anhydrous magnesium sulfate and concentrated to an oil.

Purification was accomplished by preparative HPLC (Water's Associates Prep LC/System 500, silica gel, elution with methanol). Concentration of the appropriate fractions yielded 2.5 g of 2-amino-N-methyl-α-(2-pyridinyl)benzeneethanamine as an oil.

| ANALYSIS: | | |
|---|---|---|
| Calculated for $C_{14}H_{17}N_3$: | 73.98%C | 7.54%H |
| Found: | 73.74%C | 7.54%H |

EXAMPLE 22

4,5-Dihydro-2,3-dimethyl-4-(2-pyridinyl)-3H-1,3-benzodiazepine hemihydrate

A solution of 8.00 g of 2-amino-N-methyl-α-(2-pyridinyl)benzeneethanamine and 36 ml of triethyl orthoacetate was treated with glacial acetic acid (12 ml). After refluxing for eight hours, the volatile components were removed at 70°C on a rotary evaporator. The residual oil was decanted into an ice-water mixture and basified with 10% sodium hydroxide solution. The aqueous mixture was extracted with dichloromethane, dried over anhydrous magnesium sulfate and concentrated to an oil.

Purification was accomplished by preparation HPLC (Water's Associates Prep LC/System, silica gel, eluted with 2% triethylamine in methanol). Concentration of the appropriate fractions afforded an oil which solidified on standing. The solid was recrystallized from acetonitrile to yield 2.19 g of 4,5-dihydro-2,3-dimethyl-4-(2-pyridinyl)- 3H-1,3-benzodiazepine hemihydrate, mp 123-125°C.

| ANALYSIS: | | |
|---|---|---|
| Calculated for $C_{16}H_{17}N_3 \cdot 0.5H_2O$: | 73.90%C | 6.97%H |
| Found: | 74.38%C | 6.93%N |

EXAMPLE 23

4,5-Dihydro-2-ethyl-3-methyl-4-(2-pyridinyl)-3H-1,3-benzodiazepine dihydrochloride monohydrate

A solution of 5.0 g of 2-amino-N-methyl-α-(pyridinyl)benzeneethanamine and 13.3 ml of triethyl orthoproprionate was treated with 8 ml of glacial acetic acid. After refluxing for eight hours, the volatile components were removed at 70° C on a rotary evaporator. The residual oil was decanted into an ice-water mixture and basified with 10% sodium hydroxide solution. The aqueous mixture was extracted with dichloromethane, and the organic phase was filtered, dried over anhydrous magnesium sulfate, and concentrated as an oil.

Purification was accomplished by preparative HPLC (Water's Associates Prep LC/System 500, silica gel, elution with 2% triethylamine in methanol). Concentration of the appropriate fractions afforded 4,5-dihydro-2-ethyl-3-methyl-4-(2-pyridinyl)-3H-1,3-benzodiazepine as an oil. The oil was dissolved in methanol and treated with ethereal hydrogen chloride. Dilution with diethyl ether precipitated the corresponding hydrochloride salt. The salt was collected and dried in vacuo to yield 3.18 g of 4,5-dihydro-2-ethyl-3-methyl-4-(2-pyridinyl)-3H-1,3-benzodiazepine dihydrochloride monohydrate, mp 235 - 240° C.

| ANALYSIS: | | | |
|---|---|---|---|
| Calculated for $C_{17}H_{19}N_3 \cdot 2HCl \cdot H_2O$:<br>Found: | 57.28%C<br>57.72%C | 6.50%H<br>6.32%H | 11.79%N<br>11.79%N |

## EXAMPLE 24

4,5-Dihydro-3-methyl-2-phenyl-4-(2-pyridinyl)-3H-1,3-benzodiazepine dihydrochloride hydrate (4:1)

A solution of 3.5 g of 2-amino-N-methyl-α-2-pyridinyl)benzeneethanamine and 14 ml of trimethyl orthobenzoate was treated with 8 ml of glacial acetic acid. After refluxing for eight hours, the volatile components were removed at 70° C on a rotary evaporator. The residual oil was decanted into an ice-water mixture and basified with 10% sodium hydroxide solution. The aqueous mixture was extracted with dichloromethane and the organic phase was dried over anhydrous magnesium sulfate, filtered, and concentrated to an oil.

Purification was accomplished by preparative HPLC (Water's Associates Prep LC/System 500, silica gel, elution with 2% triethylamine in methanol). Concentration of the appropriate fractions afforded an oil which was dissolved in methanol and treated with ethereal hydrogen chloride. Dilution with diethyl ether precipitated the hydrochloride salt. The salt was collected by vacuum filtration and dried in vacuo to yield 1.42 g of 4,5-dihydro-3-methyl-2-phenyl-4-(2-pyridinyl)-3H-1,3-benzodiazepine dihydrochloride hydrate (4:1), mp 244-247° C.

| ANALYSIS: | | | |
|---|---|---|---|
| Calculated for $C_{21}H_{19}N_3.2HCl.0.25H_2O$:<br>Found: | 64.51%C<br>64.46%C | 5.54%H<br>5.64%H | 10.75%N<br>10.50%N |

## EXAMPLE 25

N-Acetyl-2-amino-N-methyl-α-(2-pyridinyl)benzeneethanamine

A solution of 4.5 g of 2-amino-N-methyl-α-2-pyridinyl)benzeneethanamine in 60 ml of potassium hydroxide dried pyridine was thoroughly chilled and treated dropwise with 3.0 ml of acetic anhydride. After stirring overnight at room temperature, the reaction mixture was basified with 10% sodium hydroxide solution and extracted with dichloromethane. The organic phase was dried over magnesium sulfate, vacuum filtered, and concentrated to an oil, which solidified on standing.

The solid was recrystallized from acetonitrile and dried for 6 hours at 60°C under vacuum to yield 2.96 g of N-acetyl-2-amino-N-methyl-α-(2-pyridinyl)benzeneethanamine, mp 103-107°C.

| ANALYSIS: | | | |
|---|---|---|---|
| Calculated for $C_{16}H_{19}N_3O$: | 71.34%C | 7.11%H | 15.60%N |
| Found: | 71.01%C | 7.03%H | 15.42%N |

## EXAMPLE 26

N-[2-[2-(N-Actyl-N-methyl)amino-2-(2-pyridinyl)ethyl]-phenyl]acetamide

A chilled solution of 4.5 g of 2-amino-N-methyl-α-(2-pyridinyl)benzeneethanamine in 60 ml of potassium hydroxide dried pyridine was treated dropwise with 6 ml of acetic anhydride. After stirring overnight at room temperature, the reaction mixture was basified with 10% sodium hydroxide solution and extracted with dichloromethane. The organic phase was dried over anhydrous magnesium sulfate, vacuum filtered, and concentrated to a solid. The solid was recrystallized form acetonitrile and dried for 6 hours at 60°C under vacuum to yield 3.52 g of N-[2-[2-(N-Actyl-N-methyl)amino-2-(2-pyridinyl)ethyl]-phenyl]acetamide, mp 121-124°C.

| ANALYSIS: | | | |
|---|---|---|---|
| Calculated for $C_{18}H_{21}N_3O_2$: | 69.43%C | 6.80%H | 13.50%N |
| Found: | 69.33%C | 6.76%H | 13.50%N |

## EXAMPLE 27

N-[2-[2-(Methylamino)-2-(3-pyridinyl)ethyl]phenyl]-2,2-dimethylpropanamide

A stirred, chilled (-5°C) solution of 57.38 g of N-(2-methylphenyl)-2,2-dimethylpropanamide and 600 ml of tetrahydrofuran was treated dropwise over 1.25 hour with 264 ml of 2.5 M n-butyllithium in hexane during which time the temperature did not exceed +3°C (dry nitrogen atmosphere). The resultant solution was stirred for 35 min. with cooling and was then treated dropwise over 30 min. with a solution of 39.21 g of 3-pyridinecarboxaldehyde methylimine and 90 ml of toluene (temperature maintained below +3°C). After stirring at 0°C for 15 min. the solution was quenched by the addition of water (200 ml) and concentrated. The residue was extracted with dichloromethane (2 x 200 ml). The combined organic phase was dried over anhydrous sodium sulfate, filtered, and concentrated to an oil (117.4 g).

Purification was accomplished by preparative HPLC (Water's Associates Prep LC/System 500A, silica gel, sample applied in dichloromethane, eluted with methanol). Concentration of the appropriate fractions yielded an oil. A solution of the oil and diethyl ether was filtered, and the filtrate was concentrated to give 14.6 g of crude product. The crude product was purified by preparative HPLC (sample applied to the silica gel columns in 25 ml of dichloromethane, eluted first with ethyl acetate followed by elution with 10% methanol in dichloromethane). The appropriate fractions were combined and concentrated to an oil (10.6 g) which crystallized on trituration with hexane. The solid was isolated, washed with hexane, and dried in vacuo at ambient temperature to afford 9.24 g of N-[2-[2-(methylamino)-2-(3-pyridinyl)ethyl]phenyl]-2,2-dimethylpropanamide, mp 91.5-96°C.

| ANALYSIS: | | |
|---|---|---|
| Calculated for $C_{19}H_{25}N_3O$: | 73.28%C | 8.09%H |
| Found: | 72.75%C | 7.94%H |

EXAMPLE 28

2-Amino-N-methyl-α-(3-pyridinyl)benzeneethanamine

A stirred solution of 7.13 g of N-[2-[2-(methylamino)-2-(3-pyridinyl)ethyl]-phenyl]-2-dimethylpropanamide in 90 ml of 6N hydrochloric acid was refluxed for 5.5 hours and then allowed to stand overnight at room temperature. The solution was decanted over crushed ice, diluted with water (200 ml) and basified with 50% sodium hydroxide solution. The mixture was extracted with dichloromethane (3 x 150 ml) and the combined, organic phase was dried over anhydrous sodium sulfate, filtered, and concentrated to an oil. The oil was purified by preparative HPLC (Water's Associates Prep LC/System 500A, silica gel, sampled applied in dichloromethane, eluted with 2% triethylamine in methanol). Concentration of the appropriate fractions yielded 4.69 g of 2-amino-N-methyl-α-(3-pyridinyl)benzeneethanamine as an oil.

| ANALYSIS: | | |
|---|---|---|
| Calculated for $C_{14}H_{17}N_3$: | 73.98%C | 7.54%H |
| Found: | 73.82%C | 7.49%H |

EXAMPLE 29

4,5-Dihydro-2-ethyl-3-methyl-4-(3-pyridinyl)-3H-1,3-benzodiazepine

A stirred solution of 4.47 g of 2-amino-N-methyl-α-(3-pyridinyl)benzeneethanamine and 20.8 g of triethyl orthopropionate was treated rapidly with glacial acetic acid (4.4 ml). After refluxing for 7 hours with exclusion of moisture, the solution was concentrated on a rotary evaporator. The residual syrup was dissolved in 10% hydrochloric acid (100 ml) and the solution was extracted with diethyl ether (2 x 100 ml). The aqueous phase was basified with 10% sodium hydroxide solution and the turbid mixture was extracted with dichloromethane (2 x 100 ml). The organic phase was dried over anhydrous sodium sulfate, filtered, and concentrated to an oil.

Purification was accomplished by preparative HPLC (silica gel, sample applied in dichloromethane, eluted with 2% triethylamine in methanol). Concentration of the appropriate fractions and trituration with hexane afforded 2.51 g of 4,5-dihydro-2-ethyl-3-methyl-4-(3-pyridinyl)-3H-1,3-benzodiazepine, mp 72-75° C.

| ANALYSIS: | | |
|---|---|---|
| Calculated for $C_{17}H_{19}N_3$: | 76.95%C | 7.22%H |
| Found: | 77.16%C | 7.22%H |

EXAMPLE 30

4,5-Dihydro-2,3-dimethyl-4-(3-pyridinyl)-3H-1,3-benzodiazepine

A stirred solution of 2.5 g of 2-amino-N-methyl-α-(3-pyridinyl)benzeneethanamine and 10.71 g of triethyl orthoacetate was treated with 2.5 ml of glacial acetic acid. After refluxing for eight hours, the volatile components were removed at 70° C on a rotary evaporator. The residual syrup was dissolved in 10% hydrochloric acid (120 ml) and the solution was extracted with ether. The aqueous phase was basified with 10% sodium hydroxide solution and extracted with dichloromethane (2 x 70 ml). The combined organic phase was dried over anhydrous sodium sulfate, filtered, and concentrated to an oil.

Purification was accomplished by HPLC (Water's Associates Prep LC/System 500A, silica gel, oil applied in dichloromethane, eluted with 2% triethylamine in methanol). Concentration of the appropriate fractions yielded an oil which was dissolved in diethyl ether, filtered to remove a trace of insoluble material, and concentrated until most of the ether was removed. The residual oil was seeded with crystals obtained from a previous synthesis and dried in vacuo to give 1.32 g of 4,5-dihydro-2,3-dimethyl-4-(3-pyridinyl)-3H-1,3-benzodiazepine, mp 145-148° C.

| ANALYSIS: | | |
|---|---|---|
| Calculated for $C_{16}H_{17}N_3$: | 76.46%C | 6.82%H |
| Found: | 76.37%C | 6.78%H |

EXAMPLE 31

2-[2-(Methylamino-2-(4-pyridinyl)ethyl]phenyl]-2,2-dimethylpropanamide

A chilled solution (-5° C) of 95.65 g of 2,2-dimethyl-N-[2-[2-(methylamino)-2-(4-pyridinyl)ethyl]phenyl]-propanamide in 800 ml of tetrahydrofuran was treated dropwise over 2 hours with 440 ml of a 2.5 M solution of n-butyllithium in hexanes. After the addition was complete, the resultant solution was stirred for 2 hours with cooling, and then was treated dropwise over 29 min. with a solution of 71.0 g of 4-pyridine carboxaldehyde methylimine. The solution was stirred for an additional 20 minutes, quenched by the addition of water (350 ml), and concentrated. The residue was extracted with dichloromethane. The organic phase was dried over anhydrous magnesium sulfate, filtered, and concentrated to an oil.

Purification was accomplished by preparative HPLC (Water's Associates Prep LC/system 500A, silica gel, eluted with ethyl acetate). Concentration of the appropriate fractions yielded an oil which was further purified by preparative HPLC until free of impurities, triturated with hexane, and dried under vacuum to yield 2-[2-(methylamino-2-(4-pyridinyl)ethyl]phenyl]-2,2-dimethylpropanamide, mp 114-116° C.

| ANALYSIS: | | | |
|---|---|---|---|
| Calculated for $C_{19}H_{25}N_3O$: | 73.28%C | 8.09%H | 13.49%N |
| Found: | 73.18%C | 8.33%H | 13.77%N |

EXAMPLE 32

2-Amino-N-methyl-α-(4-pyridinyl)benzeneethanamine

A solution of 25 g of N-[2-(methylamino-2-(4-pyridinyl)ethyl]phenyl]-2,2-dimethylpropanamide in 6N hydrochloric acid (260 ml) was refluxed for 8 hours. The solution was then decanted into an ice-water mixture (800 ml), basified with 50% sodium hydroxide solution, and the mixture extracted with dichloromethane (2000 ml). The extract was dried over anhydrous magnesium sulfate and concentrated to an oil

which solidified upon standing.

Purification was accomplished by preparative HPLC (Water's Associates Prep LC/System 500A, silica gel, eluted with methanol). Concentration of the appropriate fractions yielded a solid which was triturated with hexane and dried overnight in vacuo at ambient temperature to yield 13.52 g of 2-amino-N-methyl-α-(4-pyridinyl)benzeneethanamine, mp 69-71 °C.

| ANALYSIS: | | |
|---|---|---|
| Calculated for $C_{14}H_{17}N_3$: | 73.98%C | 7.54%H |
| Found: | 73.97%C | 7.54%H |

## EXAMPLE 33

4,5-Dihydro-2,3-dimethyl-4-(4-pyridinyl)-3H-1,3-benzodiazepine

A solution of 4.0 g of 2-amino-N-methyl-α-(4-pyridinyl)benzeneethanamine and 18 ml of triethyl orthoacetate was treated with glacial acetic acid (6 ml). After refluxing for 8 hours, the volatile components were removed at 70 °C on a rotary evaporator. The residual oil was decanted into an ice-water mixture, basified with 10% sodium hydroxide solution, extracted with dichloromethane, dried over anhydrous magnesium sulfate, and concentrated to an oil.

Purification was accomplished by preparative HPLC (Water's Associates Prep LC/System 500, silica gel, eluted with triethylamine in methanol). The appropriate fractions were combined and concentrated to an oil which solidified upon standing. The solid was recrystallized from acetonitrile (10 ml) to yield 2.18 g of 4,5-dihydro-2,3-dimethyl-4-(4-pyridinyl)-3H-1,3-benzodiazepine, mp 159-161 °C.

| ANALYSIS: | | |
|---|---|---|
| Calculated for $C_{16}H_{17}N_3$: | 76.46%C | 6.82%H |
| Found: | 76.50%C | 6.59%H |

## EXAMPLE 34

4,5-Dihydro-2-ethyl-3-methyl-4-(4-pyridinyl)-3H-1,3-benzodiazepine hemihydrate

A solution of 5.00 g of 2-amino-N-methyl-α-(4-pyridinyl)benzeneethanamine and triethyl orthopropionate (24.33 ml) was treated with glacial acetic acid (7.34 ml). After refluxing for 8 hours, volatile components were removed at 40 °C on a rotary evaporator. The residual oil was acidified with 10% hydrochloric acid aqueous solution (75 ml) and extracted with diethyl ether (3 x 100 ml). The aqueous phase was basified with 10% sodium hydroxide solution and extracted with dichloromethane (3 x 100 ml). The combined dichloromethane layers were washed with water (100 ml), dried over anhydrous sodium sulfate and concentrated on a rotary evaporator to an oil.

Purification was accomplished by preparative HPLC (Water's Associates Prep LC/System 500A; silica gel; eluted with 2% triethylamine in methanol). Concentration of the appropriate fractions afforded an oil, which solidified upon refrigeration. The solid was triturated with ether, and dried in vacuo to afford 2.98 g of 4,5-dihydro-2-ethyl-3-methyl-4-(4-pyridinyl)-3H-1,3-benzodiazepine hemihydrate, mp 58-68 °C.

| ANALYSIS: | | | |
|---|---|---|---|
| Calculated for $C_{17}H_{19}N_3 \cdot 0.5H_2O$: | 74.42%C | 7.35%H | 15.32%N |
| Found: | 75.22%C | 7.22%H | 15.43%N |

## EXAMPLE 35

4,5-Dihydro-3-methyl-4-(3-methyl-2-thienyl)-2-(1-propyl)-3H-1,3-benzodiazepine hydrochloride monohydrate

A solution of 5.0 g of 2-amino-N-methyl-α-(3-methyl-2-thienyl)benzeneethanamine, 18.02 ml trimethyl orthobutyrate and 6.8 ml of glacial acetic acid was refluxed for 8 hours under a nitrogen atmosphere. The solution was concentrated at 40° C on a rotary evaporator and the residual oil was acidified with 10% hydrochloric acid solution (60 ml) and extracted with diethyl ether (3 x 70 ml). The acidic aqueous phase was basified with 10% sodium hydroxide solution (100 ml) and extracted with diethyl ether (3 x 100 ml). The ethereal phase was dried over sodium sulfate/magnesium sulfate, filtered and concentrated to an oil. Purification was accomplished by HPLC (Water's Associates Prep LC/System 500A, silica gel, utilizing methanol followed by 2% triethylamine in methanol as successive eluents). Concentration of the appropriate fractions yielded 4,5-dihydro-3-methyl-4-(3-methyl-2-thienyl)-2-(1-propyl)-3H-1,3-benzodiazepine as an oil.

The oil was treated with ethereal hydrogen chloride, and the resultant precipitate triturated with ethyl acetate to afford 2.93 g of 4,5-dihydro-3-methyl-4-(3-methyl-2-thienyl)-2-(1-propyl)-3H-1,3-benzodiazepine hydrochloride monohydrate, mp 181.5-182.0° C.

| ANALYSIS: | | | |
|---|---|---|---|
| Calculated for $C_{18}H_{22}N_2S.HCl.H_2O$: | 61.25%C | 6.57%H | 7.94%N |
| Found: | 61.60%C | 6.96%H | 8.41%N |

## EXAMPLE 36

(a) 2-Amino-5-bromo-N-methyl-α-(3-methyl-2-thienyl)-benzeneethaneamine

A stirred solution of 5.02 g of 2-amino-N-methyl-α-(3-methyl-2-thienyl)benzeneethanamine and 79 ml of dimethylformamide was treated with a solution of 4.71 g N-bromo-succinimide in 55 ml of dimethylformamide. The solution was stirred for 8 h with the exclusion of moisture and then concentrated to an oil on a rotary evaporator (70° C) under high vacuum. The oil was diluted with distilled water (100 ml) and basified with a 10% sodium hydroxide solution (25 ml). The basic solution was extracted with dichloromethane (3 x 100 ml). The organic phase was dried over anhydrous sodium sulfate, filtered and concentrated to an oil. Thin layer chromatography (silica gel, methanol eluent) indicated a 2-component mixture.

Purification was accomplished by preparative HPLC (Water's Associates Prep LC/System 500, silica gel, utilizing methanol as the eluent). Concentration of the slower eluting fractions yielded an oil which was dissolved in diethyl ether and allowed to crystallize. The resulting precipitate was collected and dried to afford 2.6 g of 2-amino-5-bromo-N-methyl-α(3-methyl-2-thienyl)benzeneethanamine, mp 68-70° C.

| ANALYSIS: | | | |
|---|---|---|---|
| Calculated for $C_{14}H_{17}BrN_2S$: | 51.70%C | 5.27%H | 8.61%N |
| Found: | 52.11%C | 5.18%H | 8.43%N |

(b) 2-Amino-3,5-dibromo-N-methyl-α-(3-methyl-2-thienyl)benzeneethanamine dihydrochloride

The faster eluting fractions obtained from the HPLC separation described supra were concentrated to an oil which was dissolved in diethyl ether, filtered free of insoluble material and concentrated. Trituration of the resultant oil with hexane afforded 2-amino-3,5-dibromo-N-methyl-α-(3-methyl-2-thienyl)-benzeneethanamine as a solid. The solid was dissolved in methanol and treated with ethereal hydrogen chloride. Dilution with diethyl ether precipitated 0.9 g of the corresponding dihydrochloride salt, mp 141 - 143° C.

| ANALYSIS: | | | |
|---|---|---|---|
| Calculated for $C_{14}H_{16}Br_2N_2S.2HCl$: | 35.25%C | 3.80%H | 5.87%N |
| Found: | 35.55%C | 3.89%H | 5.51%N |

EXAMPLE 37

N-[2-[2-(Methylamino)-2-(5-methyl-2-thienyl)ethyl]phenyl]-2,2-dimethylpropanamide hydrochloride hemihydrate

A stirred, chilled (5° C) solution of 60 g of 2,2-dimethyl-N-[(2-methyl)phenyl]propanamide and 470 ml of tetrahydrofuran was treated over one hour with 284 ml of a 2.5 M solution of n-butyllithium in hexane (nitrogen atmosphere). The solution was stirred for 45 min at 0° C and the resultant suspension was then treated over one hour with a solution of 52.41 g of 5-methyl-2-thiophenecarboxaldehyde methylimine in 470 ml of tetrahydrofuran (temperature maintained below 10° C). After stirring the mixture at room temperature for 45 minutes, 225 ml of water was added to quench the reaction. The mixture was concentrated on a rotary evaporator and the aqueous residue was extracted with diethyl ether (3 x 100 ml). The combined organic phase was dried over anhydrous sodium sulfate and magnesium sulfate, filtered, and concentrated to yield 114 g of N-[2-[2-(methylamino)-2-(5-methyl-2-thienyl)ethyl]phenyl]-2,2-dimethyl propanamide as an oil.

A 10 g aliquot of the oil was purified by preparative HPLC (Water's Associates Prep LC/System 500 A, silica gel, sample applied in dichloromethane, eluted with 25% acetone in hexane). A solution of the oil and methanol was treated with a slight excess of ethereal hydrogen chloride and then diluted with diethyl ether to yield N-[2-[2-(methylamino)-2-(5-methyl-2-thienyl)-ethyl]phenyl]-2,2-dimethylpropanamide hydrochloride hemihydrate (3.0 g), mp 91-101° C.

| ANALYSIS: | | | |
|---|---|---|---|
| Calculated for $C_{19}H_{26}N_2O.HCl.0.5H_2O$: | 60.70%C | 7.51%H | 7.45%N |
| Found: | 60.55%C | 7.45%H | 7.34%N |

EXAMPLE 38

2-Amino-N-methyl-α-(5-methyl-2-thienyl)benzeneethanamine

A solution of 22.73 g of N-[2-[2-(methylamino)-2-(5-methyl-2-thienyl)ethyl]phenyl]-2,2-dimethyl-propanamide and 100 ml of 6N hydrochloric acid solution was refluxed for eight hours. The solution was decanted over 200 ml of ice and basified with 70 ml of a 50% sodium hydroxide solution. The aqueous mixture was extracted with dichloromethane and the organic phase was dried over anhydrous sodium sulfate, filtered, and concentrated to an oil.

Purification was accomplished by preparative HPLC (Water's Associates Prep LC/System 500, silica gel, eluted with methanol). Concentration of the appropriate fractions afforded an oil which was dissolved in 100 ml of diethyl ether, filtered to remove any insoluble impurities, and concentrated to yield 10.77 g of 2-amino-N-methyl-α- (5-methyl-2-thienyl)benzeneethanamine as an oil.

| ANALYSIS: | | | |
|---|---|---|---|
| Calculated for $C_{14}H_{18}N_2S$: | 68.25%C | 7.36%H | 11.37%N |
| Found: | 68.07% | 7.20%H | 11.14%N |

## EXAMPLE 39

4,5-Dihydro-2,3-dimethyl-4-(5-methyl-2-thienyl)-3H-1,3-benzodiazepine

A stirred solution of 7.1 g of 2-amino-N-methyl-α-(5-methyl-2-thienyl)benzeneethanamine and 31.6 ml of triethyl orthoacetate was treated with 6.9 ml of glacial acetic acid. After refluxing for 8 hours the solution was concentrated at 70° C on a rotary evaporator. The resulting oil was washed with 10% hydrochloric acid solution (120 ml), followed by ether (2 x 100 ml). The aqueous phase was basified with a 10% sodium hydroxide solution (120 ml) and extracted with dichloromethane (3 x 100 ml). The combined organic phase was dried over anhydrous sodium sulfate, filtered and concentrated. The concentrate was purified by preparative HPLC (Water's Associates Prep LC/System 500, silica gel, eluted with 2% triethylamine in methanol). Concentration of the appropriate fractions yielded an oil. The oil was dissolved in diethyl ether and filtered to remove insoluble impurities. Concentration of the filtrate yielded 2.15 g of 4,5-dihydro-2,3-dimethyl-4-(5-methyl-2-thienyl)-3H-1,3-benzodiazepine, mp 139-140° C.

| ANALYSIS: | | | |
|---|---|---|---|
| Calculated for $C_{16}H_{18}N_2S$: | 71.07%C | 6.71%H | 10.36%N |
| Found: | 71.06%C | 6.80% | 10.22%N |

## EXAMPLE 40

4,5-Dihydro-2-ethyl-3-methyl-4-(5-methyl-2-thienyl)-3H-1,3-benzodiazepine

A stirred solution of 5.88 g of 2-amino-N-methyl-α-(5-methyl-2-thienyl)benzeneethanamine and 29 ml of triethyl orthopropionate was treated with 5.5 ml of glacial acetic acid. After refluxing for 8 hours, the solution was concentrated to an oil at 70° C on a rotary evaporator. The oil was washed with a 10% hydrochloric acid solution (100 ml) and water was added to dissolve observed solids. The aqueous phase was washed with diethyl ether (2 x 50 ml) and then basified with a 10% sodium hydroxide solution (100 ml). The mixture was extracted with dichloromethane (3 x 100 ml) and the combined organic phase was dried over anhydrous sodium sulfate, filtered, and concentrated.

Purification was accomplished by preparative HPLC (Water's Associates Prep LC/System 500, silica gel, 2% triethylamine in methanol as the eluent). Concentration of the appropriate fractions yielded an oil which was dissolved in ether and filtered to remove all traces of insoluble material. The filtrate was concentrated to an oil, which was triturated with hexane to afford 1.51 g of 4,5-dihydro-2-ethyl-3-methyl-4-(5-methyl-2-thienyl)- 3H-1,3-benzodiazepine as a solid, mp 66° C.

| ANALYSIS: | | | |
|---|---|---|---|
| Calculated for $C_{17}H_{20}N_2S$: | 71.79%C | 7.09%H | 9.85%N |
| Found: | 71.74%C | 7.21%H | 9.90%N |

## EXAMPLE 41

2-[(2,5-Dioxo-1-pyrrolidinyl)methyl]amino-N-formyl-N-methyl-α-(3-methyl-2-thienyl)benzeneethanamine

A stirred solution of 8 g of 2-amino-N-formyl-N-methyl-α-(3-methyl-2-thienyl)benzeneethanamine in 30 ml of 95% ethanol was treated with 2.4 ml of a 37% formaldehyde solution and 3.6 g of succinimide. The solution was heated for 6 hours at 98°C and then allowed to stand at ambient temperature for 24 hours. The resulting precipitate was filtered, washed (95% ethanol; 3 x 50 ml), and dried in vacuo (40°C) to afford 7.62 g of 2-[(2,5-dioxo-1-pyrrolidinyl)methyl]amino-N-formyl-N-methyl-α-(3-methyl-2-thienyl)-benzeneethanamine, mp 160-164°C.

| ANALYSIS: | | | |
|---|---|---|---|
| Calculated for $C_{20}H_{23}N_3O_3S$: | 62.30%C | 6.01%H | 10.90%N |
| Found: | 62.09%C | 5.98%H | 10.82%N |

## EXAMPLE 42

N-Formyl-N-methyl-2-methylamino-α-(3-methyl-2-thienyl)benzeneethanamine hydrochloride monohydrate

A mixture of 6.39 g of 2-[(2,5-dioxo-1-pyrrolidinyl) methyl]amino-N-formyl-N-methyl-α-(3-methyl-2-thienyl)benzeneethanamine, 20 ml of anhydrous dimethyl sulfoxide and 0.72 g of sodium borohydride was stirred at ambient temperature for 15 minutes and then heated on a steam bath for 15 minutes with occasional agitation. After standing for 2 hours at ambient temperature, the reaction mixture was decanted into 150 ml of water and extracted with diethyl ether (2 x 100 ml). The organic phase was washed with water (2 x 100 ml), dried over anhydrous sodium sulfate, and concentrated to an oil. Thin layer chromatography of the resultant oil indicated a multi-component mixture. Purification of the mixture was accomplished by HPLC (Waters Associates Prep LC/System 500, silica gel, elution with methanol]. Concentration of the appropriate fractions afforded N-formyl-N-methyl-2-methylamino-α-(3-methyl-2-thienyl)-benzeneethanamine) as an oil.

A solution of the oil and diethyl ether was treated with ethereal hydrogen chloride to precipitate the corresponding hydrochloride salt. The precipitate was dried in vacuo to yield 1.7 g of N-formyl-N-methyl-2-methylamino-α-(3-methyl-2-thienyl)-benzeneethanamine hydrochloride monohydrate, mp 140-145°C.

| ANALYSIS: | | | |
|---|---|---|---|
| Calculated for $C_{16}H_{20}N_2OS \cdot HCl \cdot H_2O$: | 56.05%C | 6.76%H | 8.17%N |
| Found: | 56.00%C | 6.79%H | 7.85%N |

## EXAMPLE 43

2-Amino-5-chloro-N-methyl-α-(3-methyl-2-thienyl)benzeneethanamine

A stirred solution of 8 g of 2-amino-N-methyl-α-(3-methyl-2-thienyl)benzeneethanamine in 40 ml of N,N-dimethylformamide was treated with a solution of 6.41 g of N-chlorosuccinimide in 40 ml of N,N-dimethylformamide and then stirred at room temperature for 8 hours. Evaporation of the volatiles afforded an oil which was decanted into 100 ml of water, basified with 30 ml of 10% aqueous sodium hydroxide and extracted with dichloromethane (2 x 100 ml). The extract was washed with water (2 x 100 ml), dried over anhydrous sodium sulfate and concentrated. Thin layer chromatography of the resultant oil indicated a multicomponent mixture. Purification of the mixture was accomplished by successive HPLC separations (Water's Associates Prep LC/System 500, silica gel, elution with methanol). Concentration of the appropriate fractions afforded a residue which was dissolved in diethyl ether, filtered free of insolubles and concentrated. The resultant oil crystallized upon standing to yield 1.05 g of 2-amino-5-chloro-N-methyl-α-(3-methyl-2-thienyl)benzeneethanamine, mp 59-61 °C.

| ANALYSIS: | | | |
|---|---|---|---|
| Calculated for $C_{14}H_{17}ClN_2S$: | 59.89%C | 6.10%H | 9.97%N |
| Found: | 60.24%C | 6.10%H | 9.99%N |

EXAMPLE 44

N-[2-(2-(methylamino)-2-(2-methyl-3-thienyl)ethyl)phenyl]-2,2-dimethylpropanamide

A chilled solution (-2 °C) of 37.9 g of 2,2-dimethyl-N-[(2-methyl)phenyl]propanamide in 350 ml of tetrahydrofuran was treated dropwise over 2 hours with 180 ml of 2.5 m n-butyllithium in hexane, and then stirred for 45 minutes with cooling. The resultant suspension was treated dropwise over 15 minutes with a solution of 13.6 g of 2-methyl-3-thiophenecarboxaldehyde methylimine in 40 ml of tetrahydrofuran. The reaction mixture was stirred for 1 hour, quenched by the addition of 17.4 ml of methanol and 200 ml of water, and concentrated. The concentrate was extracted with dichloromethane and the combined extract was dried over anhydrous sodium sulfate, filtered, and evaporated to an oil. Preliminary purification of the oil was achieved by HPLC separations (Water's Associates Prep LC/System 500, silica, elution with 20% methanol in ethyl acetate). Concentration of the appropriate fractions afforded an oil which was dissolved in diethyl ether and concentrated to yield 24.0 g of N-[2-(2-(methylamino)-2-(2-methyl-3-thienyl)ethyl)phenyl]-2,2-dimethylpropanamide, mp 95-96 °C.

| ANALYSIS: | | | |
|---|---|---|---|
| Calculated for $C_{19}H_{26}N_2OS$: | 69.05%C | 7.93%H | 8.48%N |
| Found: | 68.97%C | 7.95%H | 8.41%N |

EXAMPLE 45

2-Amino-N-methyl-α-(2-methyl-3-thienyl)benzeneethanamine dihydrochloride

A solution of 11.0 g of N-[2-(2-(methylamino)-2-(2-methyl-3-thienyl)ethyl)phenyl]-2,2-dimethylpropanamide in 300 ml of 6 N hydrochloric acid was refluxed for 8 hours. The solution was then decanted into an ice-water mixture and basified with 50% aqueous sodium hydroxide solution. The aqueous mixture was extracted with dichloromethane (900 ml) and the organic phase was dried over anhydrous sodium sulfate, filtered and concentrated to yield 9.5 g of 2-amino-N-methyl-α-(2-methyl-3-thienyl)-

benzeneethanamine as an oil.

A 3.0 g aliquot of the oil was dissolved in 45 ml of methanol and the resultant solution was acidified to a pH of 1 with ethereal hydrogen chloride. Dilution with anhydrous diethyl ether (45 ml) precipitated the corresponding hydrochloride salt.

The precipitate was dried in vacuo (40°) over sodium hydroxide to yield 2.5 g of 2-amino-N-methyl-α-(2-methyl-3-thienyl)benzeneethanamine dihydrochloride, mp 229-230° C.

| ANALYSIS: | | | |
|---|---|---|---|
| Calculated for $C_{14}H_{18}N_2S \cdot 2HCl$: | 52.66%C | 6.31%H | 8.78%N |
| Found: | 52.40%C | 6.29%H | 8.59%N |

EXAMPLE 46

N-Acetyl-2-amino-N-methyl-α-(3-methyl-2-thienyl)benzeneethanamine

A stirred, ice water chilled solution of 5.07 g of 2-amino-N-methyl-α-(3-methyl-2-thienyl)-benzeneethanamine in 40 ml of pyridine was treated dropwise with 2.25 g of acetic anhydride. The solution was stirred with cooling for 15 minutes and then at room temperature for 3 hours. After standing overnight, the reaction mixture was decanted into water (200 ml), treated with dichloromethane (100 ml) and basified with 10% sodium hydroxide solution. The aqueous phase was extracted with dichloromethane (150 ml), and the combined organic phase was filtered and concentrated. The resulting solid was stirred with toluene and evaporated to dryness. Recrystallization from propionitrile (14 ml) afforded 3.0 g of N-acetyl-2-amino-N-methyl-α-(3-methyl-2-thienyl)benzeneethanamine, mp 176-181.5° C.

| ANALYSIS: | | | |
|---|---|---|---|
| Calculated for $C_{16}H_{20}N_2OS$: | 66.63%C | 6.99%H | 9.71%N |
| Found: | 66.82%C | 7.00%H | 9.73%N |

EXAMPLE 47

(a) 2-Amino-N-formyl-N-methyl-α-(3-methyl-2-thienyl)benzeneethanamine

A stirred solution of 5.00 g of 2-amino-N-methyl-α-(3-methyl-2-thienyl)benzeneethanamine and 235 ml of ethyl formate was refluxed for 8 hours. Volatile components were removed at 60° C on a rotary evaporator. Thin layer chromatography (silica gel, ethyl acetate as the eluent) of the resultant oil indicated a multi-component mixture. HPLC of the mixture (Water's Associates Prep LC/System 500, silica gel, elution with ethyl acetate) permitted component separation. The faster eluting fractions were concentrated to an oil which crystallized upon standing. Recrystallization from methanol-water afforded 1.2 g of 2-amino-N-formyl-N-methyl-α-(3-methyl-2-thienyl)benzeneethanamine, mp 111-112° C.

| ANALYSIS: | | | |
|---|---|---|---|
| Calculated for $C_{15}H_{18}N_2OS$: | 65.66%C | 6.61%H | 10.21%N |
| Found: | 65.46%C | 6.66%H | 10.26%N |

(b) N-[2-[2-(N-Formyl-N-methyl)amino-2-(3-methyl-2-thienyl)ethyl]phenylformamide

Concentration of the more slowly eluting fractions obtained from the HPLC separation described supra afforded a residue which was dissolved in diethyl ether, filtered, and concentrated to an oil which solidified upon standing. The crystalline product was triturated in hexane, filtered, and dried in vacuo at 40°C to afford 10 g of N-[2-[2-(N-formyl-N-methyl)amino-2-(3-methyl-2-thienyl)ethyl]phenyl]formamide, mp 128-130°C.

| ANALYSIS: | | | |
|---|---|---|---|
| Calculated for $C_{16}H_{18}N_2O_2S$: | 63.56%C | 6.00%H | 9.27%N |
| Found: | 63.34%C | 6.06%H | 9.18%N |

EXAMPLE 48

2-Amino-N,N-dimethyl-α-(3-methyl-2-thienyl)benzeneethanamine

A stirred solution of 5.79 g of 2-amino-N-formyl-N-methyl-α-(3-methyl-2-thienyl)benzeneethanamine in 400 ml of tetrahydrofuran was treated dropwise under nitrogen with 90 ml of a 1 M solution of borane in tetrahydrofuran. The solution was stirred overnight at room temperature and then quenched with 60 ml of a 10% sodium hydroxide solution. The aqueous mixture was extracted with dichloromethane (2 x 100 ml), dried over anhydrous sodium sulfate, filtered and concentrated. The concentrate was treated with 15 ml of glacial acetic acid and 35 ml of concentrated hydrochloric acid. After stirring at room temperature overnight, the solution was decanted over 200 g of ice, basified with 20 ml of 50% aqueous sodium hydroxide, and extracted with dichloromethane (3 x 100 ml). The organic phase was dried over anhydrous sodium sulfate, filtered and concentrated. Purification was accomplished by means of HPLC (Water's Associates Prep LC/System 500, silica gel, elution with methanol). Concentration of the appropriate fraction afforded a solid which was recrystallized from diethyl ether to afford 3.7 g of 2-amino-N,N-dimethyl-α-(3-methyl-2-thienyl)-benzeneethanamine, mp 75-77°C.

| ANALYSIS: | | | |
|---|---|---|---|
| Calculated for $C_{15}H_{20}N_2S$: | 69.19%C | 7.74%H | 10.76%N |
| Found: | 69.25%C | 7.81%H | 10.66%N |

EXAMPLE 49

2-Amino-5-bromo-N-methyl-α-(2-methyl-3-thienyl)benzeneethanamine

A stirred solution of 3.0 g of 2-amino-N-methyl-α-(2-methyl-3-thienyl)benzeneethanamine in 50 ml of N,N-dimethylformamide was treated with a solution of 2.6 g of N-bromosuccinimide in 50 ml of N,N-dimethylformamide and allowed to stand overnight at room temperature. Evaporation of the volatiles afforded an oil which was purified by HPLC (Water's Associates Prep LC/System 500, silica gel, elution with methanol). The resultant oil was dissolved in diethyl ether, filtered and concentrated. Trituration with diethyl ether afforded 1.2 g of 2-amino-5-bromo-N-methyl-α-(2-methyl-3- thienyl)benzeneethanamine, mp 72.5-74°C.

| ANALYSIS: | | | |
|---|---|---|---|
| Calculated for $C_{14}H_{17}BrN_2S$: | 51.69%C | 5.27%H | 8.61%N |
| Found: | 52.10%C | 5.25%H | 8.57%N |

## EXAMPLE 50

N-Methyl-2 methylamino-α-(3-methyl-2-thienyl)benzeneethanamine dihydrochloride

N-Formyl-N-methyl-2-methylamino-α-(3-methyl-2-thienyl)benzeneethanamine (4.1 g) was treated with 18 ml of 3N hydrochloric acid. After refluxing for 1.5 hours the solution was decanted over ice, basified with 12 ml of a 50% aqueous sodium hydroxide solution and extracted with dichloromethane (3 x 60 ml). The organic phase was dried over anhydrous sodium sulfate and concentrated to an oil. Purification of the oil was accomplished by HPLC (Water's Associates Prep LC/System 500, silica gel, elution with methanol). Concentration of the appropriate fractions afforded a residue which was dissolved in diethyl ether, filtered free of insolubles, and concentrated to afford N-methyl-2-methylamino-α-(3-methyl-2-thienyl)-benzeneethanamine as an oil. The oil was dissolved in methanol and treated with ethereal hydrogen chloride. Dilution with diethyl ether precipitated 2.4 g of the corresponding dihydrochoride salt, mp 191-192° C.

| ANALYSIS: | | | |
|---|---|---|---|
| Calculated for $C_{15}H_{20}N_2S \cdot 2HCl$: | 54.05%C | 6.65%H | 8.40%N |
| Found: | 54.19%C | 6.64%H | 8.36%N |

## EXAMPLE 51

N,N-Dimethyl-2-methylamino-α-(3-methyl-2-thienyl)benzeneethanamine

A solution of 7.2 g of N-[2-[2-(N-formyl-N-methyl)amino-2-(3-methyl-2-thienyl)ethyl]phenyl]formamide in 600 ml of tetrahydrofuran was treated with 144 ml of a 1 M borane-tetrahydrofuran complex under nitrogen at room temperature. After stirring overnight, the mixture was quenched with 10% sodium hydroxide solution, and concentrated to remove the solvent. The concentrate was extracted with dichloromethane (3 x 150 ml), dried over anhydrous sodium sulfate, filtered, and concentrated to an oil. The oil was dissolved in 20 ml of glacial acetic acid, and treated dropwise with 120 ml of concentrated hydrochloric acid, and left to stir at room temperature overnight. The solution was then decanted over 300 g of ice, basified with 50% sodium hydroxide solution, extracted with dichloromethane (3 x 200 ml), dried over anhydrous sodium sulfate, filtered and concentrated to an oil.

Purification of the oil was accomplished by preparative HPLC (Water's Associates Prep LC/System 500, silica gel, elution with methanol). The appropriate fractions were collected, combined and concentrated to give 4.6 g of N,N-dimethyl-2-methylamino-α-(3-methyl-2-thienyl)benzeneethanamine as an oil.

| ANALYSIS: | | | |
|---|---|---|---|
| Calculated for $C_{16}H_{22}N_2S$: | 70.03%C | 8.08%H | 10.21%N |
| Found: | 70.13%C | 8.23%H | 10.10%N |

## EXAMPLE 52

N,N-Dimethyl-2-dimethylamino-α-(3-methyl)-2-thienyl)benzeneethanamine

A solution of 5.4 g of 2-amino-N-methyl-α-(3-methyl-2-thienyl)benzeneethanamine in 50 ml of acetonitrile and 11.5 ml of a 37% (w/w) formaldehyde solution was treated with 3.1 g of sodium cyanoborohydride. After stirring at room temperature for 2.5 hours, the reaction mixture was dissolved in 80 ml of diethyl ether and extracted with an aqueous potassium hydroxide solution (3 x 75 ml). The ethereal layer was washed with 150 ml of saturated brine, dried with anhydrous potassium carbonate, filtered and concentrated.

Purification of the resultant oil was accomplished by preparative HPLC (Water's Associates Prep LC/System 500, silica gel, elution with methanol). The appropriate fractions were collected, combined and concentrated. The concentrate was dissolved in diethyl ether, filtered and concentrated to afford 3.1 g of N,N-dimethyl-2-dimethylamino-α-(3-methyl-2-thienyl)benzeneethanamine as an oil.

| ANALYSIS: | | | |
|---|---|---|---|
| Calculated for $C_{17}H_{24}N_2S$: | 70.79%C | 8.40%H | 9.71%N |
| Found: | 70.70%C | 8.41%H | 9.79%N |

## EXAMPLE 53

N-Formyl-2-hydroxy-N-methyl-α-(3-methyl-2-thienyl)benzeneethanamine

A stirred suspension of 8.87 g of 2-amino-N-formyl-N-methyl-α-(3-methyl-2-thienyl)benzeneethanamine and 71 ml of 50% (w/v) sulfuric acid was chilled with an ice water bath for 15 minutes. The suspension was treated dropwise over one minute with a solution of sodium nitrite (2.45 g) and water (13 ml). After stirring with chilling for 15 minutes, the mixture was strained through glass wool into a dropping funnel and then added over a few minutes to a refluxing solution of 63.84 g of copper sulfate and 130 ml of water. After a few minutes of vigorous stirring, the reaction mixture was cooled to room temperature with an ice bath, diluted with water and dichloromethane and transferred to a separatory funnel. The phases were separated and the aqueous phase was extracted with dichloromethane (2 x 200 ml). The combined organic phase was dried over anhydrous sodium sulfate, filtered, and concentrated to an oil. The oil was purified by means of preparative HPLC (Water's Associates Prep LC/System 500, silica gel, sample applied in dichloromethane and eluted with ethyl acetate-hexane (1:1)). The appropriate fractions were combined and concentrated to an oil which crystallized on refrigerated storage overnight. Recrystallization from ethyl acetate (20 ml) afforded 1.62 g of N-formyl-2-hydroxy-N-methyl-α-(3-methyl-2-thienyl)benzeneethanamine, mp 142-144° C.

| ANALYSIS: | | | |
|---|---|---|---|
| Calculated for $C_{15}H_{17}NO_2S$: | 65.43%C | 6.22%H | 5.09%N |
| Found: | 65.37%C | 6.20%H | 5.00%N |

## EXAMPLE 54

2-Hydroxy-N-methyl-α-(3-methyl-2-thienyl)benzeneethanamine hydrochloride

A stirred suspension of 4.0 g of N-formyl-2-hydroxy-N-methyl-α-(3-methyl-2-thienyl)benzeneethanamine

and 40 ml of methanol was treated with 40 ml of 3N hydrochloric acid to afford a turbid yellow mixture. After heating to reflux, sufficient methanol (40 ml) was added to give a yellow solution. After refluxing for 5 hours, the solution was concentrated on a rotary evaporator to remove the methanol. The residual oil-water mixture was diluted with dichloromethane and basified with 5% sodium bicarbonate solution. The phases were separated and the aqueous phase was extracted with dichloromethane (2 x 100 ml). The combined organic phase was dried over sodium sulfate and concentrated to an oil which was shown to be three component mixture by thin layer analysis.

The oil was purified by preparative HPLC (Water's Associates Prep LC/System 500, silica gel, sample applied in dichloromethane (20 ml), elution with methanol-ethyl acetate dicholoromethane). Concentration of the appropriate fractions afforded an oil, which was repurified by preparative HPLC to remove a trace of the starting amide. Concentration of the appropriate fractions gave 2.35 g of 2-hydroxy-N-methyl-α-(3-methyl-2-thienyl)benzeneethanamine as an oil. The oil was dissolved in anhydrous ether and the solution was treated with ethereal hydrogen chloride to give a precipitate, which was collected and dried in vacuo at 40° C, over sodium hydroxide pellets. Recrystallization from absolute ethanol (20 ml) by gradual dilution with diethyl ether gave 1.54 g (37%) of 2-hydroxy-N-methyl-α-(3-methyl-2-thienyl)benzeneethanamine hydrochloride, mp 195.5-196.5° C.

| ANALYSIS: | | | |
|---|---|---|---|
| Calculated for $C_{14}H_{17}NOS \cdot HCl$: | 59.25%C | 6.39%H | 4.94%N |
| Found: | 59.24%C | 6.38%H | 4.88%N |

## EXAMPLE 55

4,5-Dihydro-2-ethyl-3-methyl-4-(2-methyl-3-thienyl)-3H-1,3-benzodiazepine

A solution of 3.95 g of 2-amino-N-methyl-α-(2-methyl-3-thienyl)benzeneethanamine, 5 ml of glacial acetic acid and 17.0 g of triethylorthopropionate was refluxed for 8 hours (under a nitrogen atmosphere). The solution was concentrated in vacuo at 80° C and the residual oil dissolved in diethyl ether (50 ml) and acidified with 10% hydrochloric acid solution. The aqueous phase was washed with diethyl ether (50 ml), basified, and extracted with dichloromethane (2 x 100 ml). The combined organic phase was dried over anhydrous sodium sulfate, filtered and concentrated. The resultant oil was purified by preparative HPLC (Water's Associates Prep LC/System 500, silica gel, elution with methanol followed by 2% triethylamine in methanol). Concentration of the appropriate fractions afforded 2.26 g of 4,5-dihydro-2-ethyl-3-methyl-4-(2-methyl-3-thienyl)-3H-1,3-benzediazepine as an oil. Trituration with hexane afforded a solid which was combined with product obtained from previous run to afford the analytical sample, mp 82-83.5° C.

| ANALYSIS: | | | |
|---|---|---|---|
| Calculated for $C_{17}H_{20}N_2S$: | 71.79%C | 7.09%H | 9.85%N |
| Found: | 71.68%C | 7.19%H | 9.75%N |

Reaction Scheme A

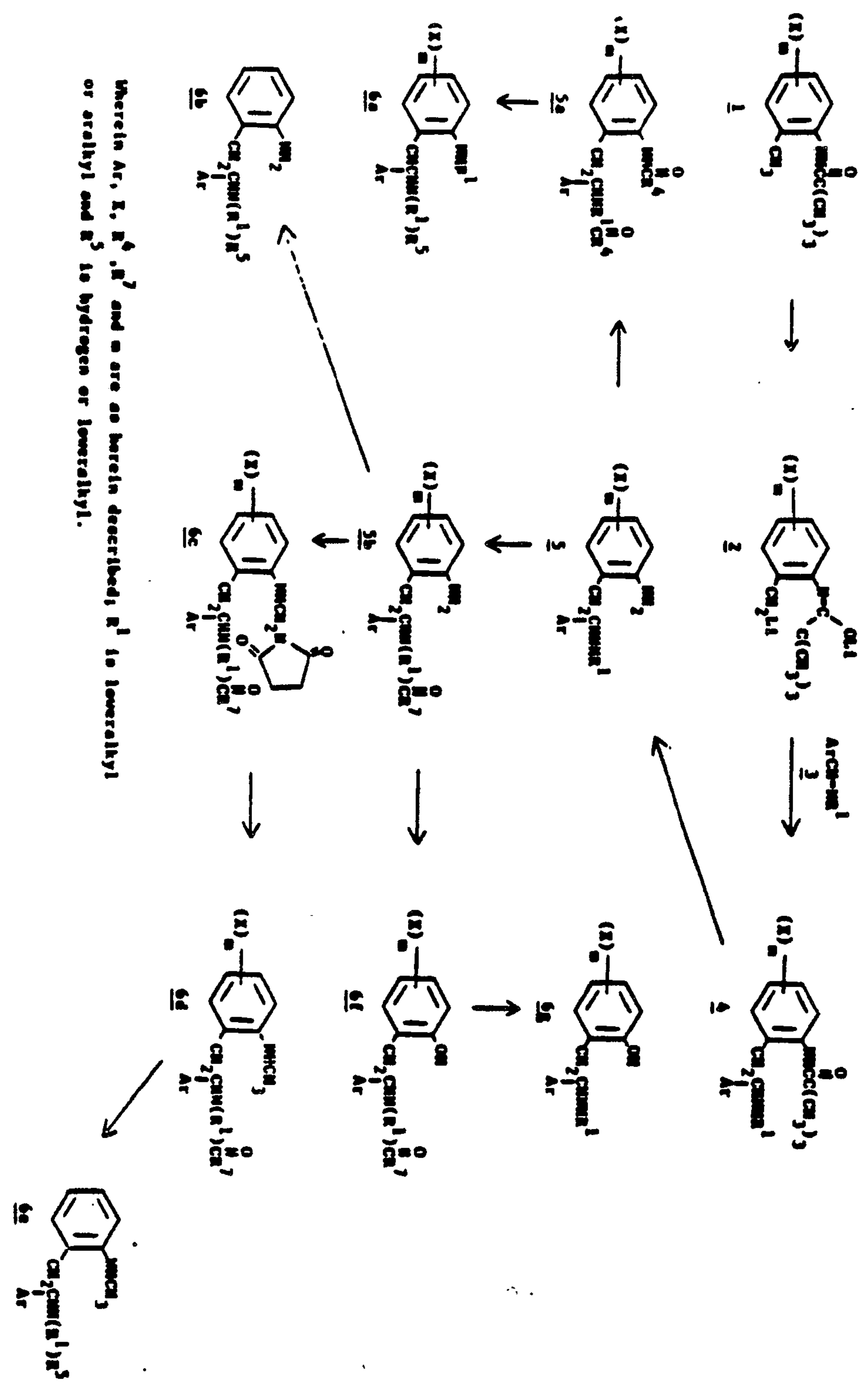

Reaction Scheme B

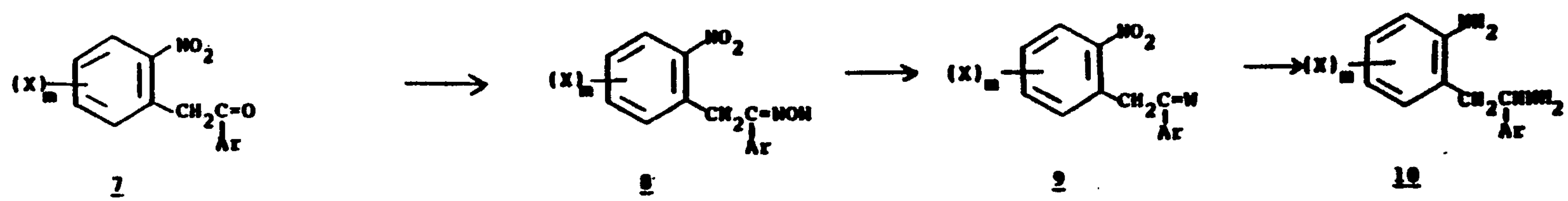

7 8 9 10

Wherein Ar, X, and m are as herein described; and W is $NOR^8$ wherein $R^8$ is an alkanoyl of from 1 to 5 carbon atoms.

Reaction Scheme C

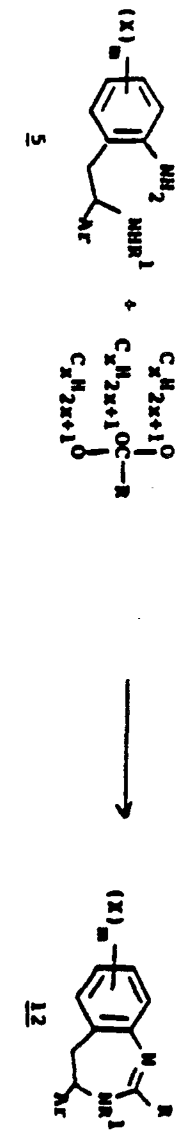

5

12

wherein X, Ar, R and m are as previously described; $R^1$ is hydrogen, loweralkyl or aralkyl and x is an integer having a value of 1 or 2.

## Claims

1. A compound of the formula I

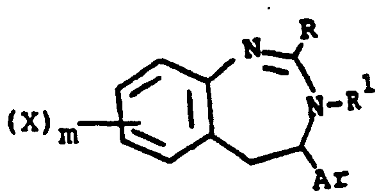

I

wherein Ar is a radical of the following formulae of

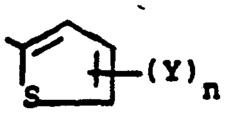, 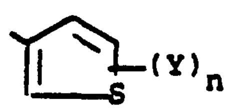, 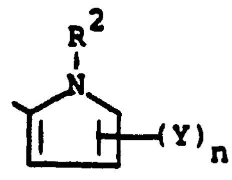, 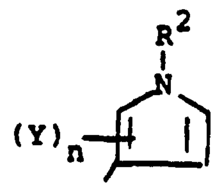

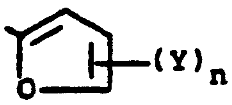, 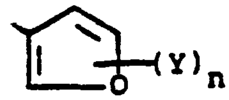,

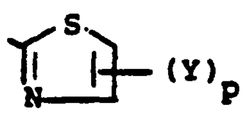, 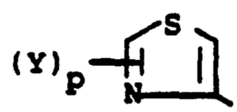, 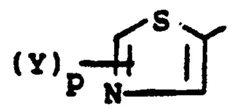,

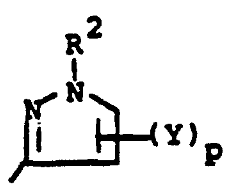, 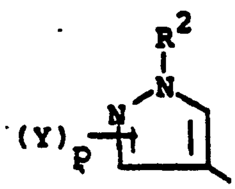, 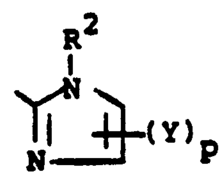,

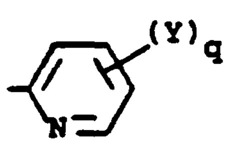, 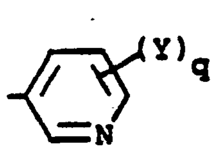, or 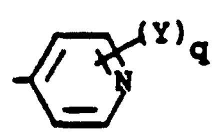

wherein $R^2$ is hydrogen, loweralkyl or loweralkanoyl, Y is halogen, hydroxyl, loweralkyl, loweralkoxy, or trifluoromethyl, n is an integer having a value from 0 to 3 inclusive, p is an integer having a value of 0 or 1, and q is an integer having a value from 0 to 4 inclusive; X is halogen, hydroxyl, nitro, loweralkyl, loweralkoxy, or trifluoromethyl; m is an integer having a value from 0 to 2 inclusive; R is hydrogen, loweralkyl, aryl, aralkyl, cycloalkylloweralkyl, loweralkenyl, or loweralkynyl; and $R^1$ is hydrogen, loweralkyl, or aralkyl, wherein for each value of m, n, p, or q each X or Y may be the same or different; the optical antipodes, geometrical isomers and pharmaceutically acceptable acid addition salts thereof.

2. A compound as defined in claim 1 wherein $R^1$ is methyl, m is 0 or 1 and Ar is a radical of the formulae

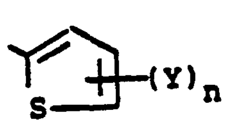, 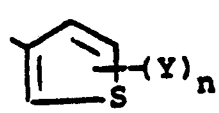,

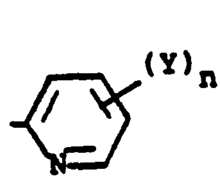, 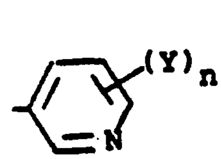, or 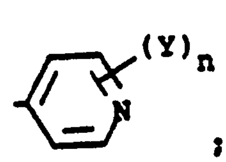;

wherein Y is loweralkyl and n is an integer having a value of 0 or 1.

3. A compound as defined in claim 2 wherein R is loweralkyl.

4. The compound as defined in claim 3 which is 4,5-dihydro-2-ethyl-3-methyl-4-(2-thienyl)-3H-1,3-benzodiazepine, or a pharmaceutically acceptable acid addition salt thereof.

5. The compound as defined in claim 3 which is 4,5-dihydro-2-ethyl-3-methyl-4-(3-methyl-2-thienyl)-3H-1,3-benzodiazepine, or a pharmaceutically acceptable acid addition salt thereof.

6. A pharmaceutical composition which comprises an effective amount of a compound as defined in claim 1 and a suitable carrier therefor.

7. Use of a compound as defined in claim 1 for the preparation of a medicament having antidepressant activity.

8. A compound of the formula II

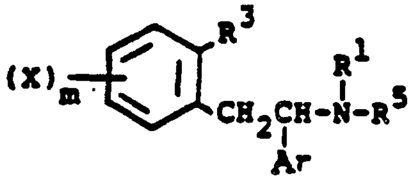

II

wherein Ar is a radical of the formulae

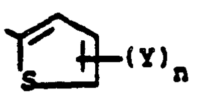 , 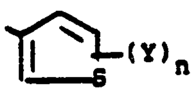 , 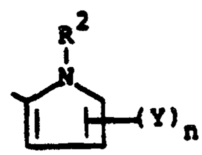 , 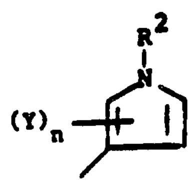

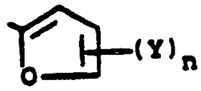 , 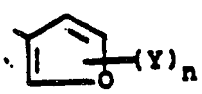 ,

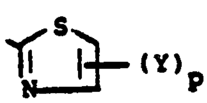 , 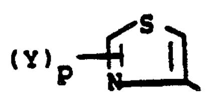 , 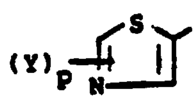 ,

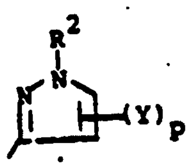 , 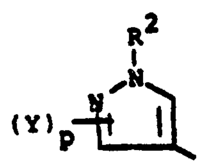 , 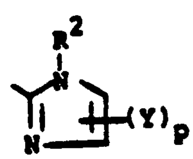 ,

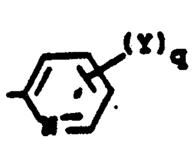 , 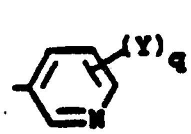 , or 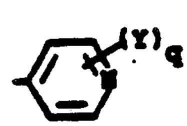

wherein $R^2$ is hydrogen, loweralkyl or loweralkanoyl, Y is halogen, hydroxyl, loweralkyl, loweralkoxy or trifluoromethyl, n is an integer having a value from 0 to 3 inclusive, p is an integer having a value of 0 or 1, and q is an integer having a value from 0 to 4 inclusive; X is halogen, hydroxyl, nitro, loweralkyl, loweralkoxy, or trifluoromethyl; m is an integer having a value from 0 to 3 inclusive; $R^1$ is hydrogen, loweralkyl, or aralkyl; $R^3$ is hydroxy, loweralkoxy, amino, loweralkylamino, diloweralkylamino or $NR^6C(O)R^4$ wherein $R^4$ and $R^6$ are independently hydrogen or loweralkyl; and $R^5$ is hydrogen, loweralkyl or $C(O)R^7$ where $R^7$ is hydrogen or loweralkyl; wherein for each value of m, n, p or q each X or Y may be the same or

different; the geometrical isomers, optical antipodes and pharmaceutically acceptable acid addition salts thereof.

9. A compound as defined in claim 8 wherein $R^3$ is amino, m is 0 or 1 and Ar is a radical of the formulae

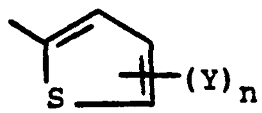 or 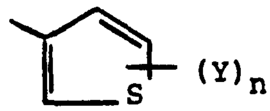

wherein Y is loweralkyl and n is an integer having a value of 0 or 1.

10. the compound as defined in claim 9 which is 2-amino-N-methyl-α-(3-methyl-2-thienyl)-benzeneethanamine or a pharmaceutically acceptable acid addition salt thereof.

11. The compound as defined in claim 9 which is 2-amino-5-bromo-N-methyl-α-(3-methyl-2-thienyl)-benzeneethanamine or a pharmaceutically acceptable acid addition salt thereof.

12. A pharmaceutical composition which comprises an effective amount of a compound as defined in claim 8 as the active ingredient and a suitable carrier therefor.

13. Use of a compound as defined in claim 8 for the preparation of a medicament having anticonvulsant activity.

14. A process for the preparation of a compound as defined in claim 1, which comprises reacting a compound of the formula 5

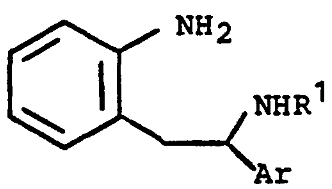

5

wherein $R^1$ and Ar are as defined in claim 3, with a compound of the formula

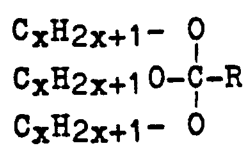

wherein R is as defined in claim 1 and x is an integer having a value of 1 or 2.

15. A process for the preparation of a compound as defined in claim 8, which comprises

a) reacting a compound of the formula 2

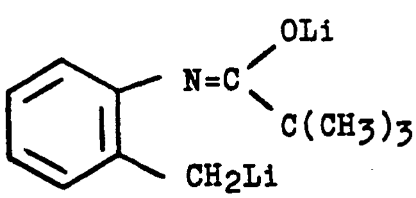

2

where X and m are as defined, with a compound of the formula $ArCH=NR^1$ where Ar and $R^1$ are as defined in claim 8, to afford a compound of the formula 4

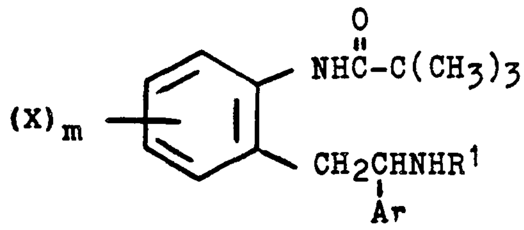

b) hydrolyzing the compound obtained to afford a compound of the formula II, where $R^3$ is amino, $R^1$ and Ar are as defined and $R^5$ is hydrogen,

c) optionally reacting a compound of the formula II, wherein $R^3$ is amino, $R^5$ is hydrogen and $R^1$ and Ar are as defined, with an alcanoic acid anhydride to afford a compound of the formula II, wherein $R^3$ is amino or the group $-NR^6C(O)R^4$, where $R^6$ is hydrogen and $R^4$ is hydrogen or loweralkyl, $R^5$ is the group $-\overset{O}{\overset{\|}{C}}-R^7$ where $R^7$ is hydrogen or loweralkyl, and $R^1$ and Ar are as defined,

d) optionally reducing a compound as obtained in step c) to afford a compound of the formula II, where $R^3$ is the group $-NHR'$ where $R'$ is hydrogen or loweralkyl, $R^5$ is loweralkyl and $R^1$ and Ar are as defined,

e) optionally treating a compound of the formula II wherein $R^3$ is amino, $R^5$ is the group $-\overset{O}{\overset{\|}{C}}-R^7$ where $R^7$ is hydrogen or loweralkyl and $R^1$ and Ar are as defined, with formaldehyde and succinimide followed by reduction of the resultant dioxo-pyrrolidinyl substituted compound of the formula 6c

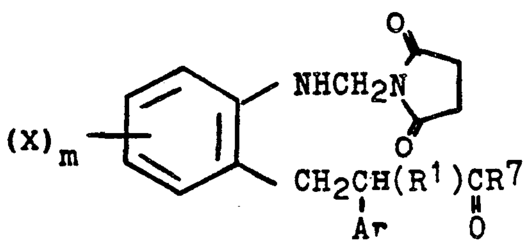

6c

to afford a compound of the formula II where $R^3$ is $-NHCH_3$ $R^5$ is the group $-\overset{O}{\overset{\|}{C}}R^7$, where $R^7$ is hydrogen or loweralkyl, and $R^1$ and Ar are as defined,

f) optionally hydrolyzing the compound as obtained in step c) to afford a compound of the formula II where $R^3$ is $-NHCH_3$, $R^5$ is hydrogen or loweralkyl and $R^1$ and Ar are as defined,

g) optionally diazotizing a compound of the formula II, where $R^3$ is amino, $R^1$ is loweralkyl, $R^5$ is the group $-\overset{O}{\overset{\|}{C}}R^7$ where $R^7$ is hydrogen or loweralkyl and Ar is as defined above, followed by quenching the resultant compound into aqueous copper sulfate solution, to afford a compound of the formula II, where $R^3$ is hydroxy, $R^1$ is loweralkyl, $R^5$ is the group $-\overset{O}{\overset{\|}{C}}R^7$ where $R^7$ is hydrogen or loweralkyl and Ar is as defined above,

h) optionally hydrolyzing a compound as obtained in step g) to afford a compound of the formula II where $R^3$ is hydroxy, $R^1$ is loweralkyl, $R^5$ is hydrogen and Ar is as defined, or

i) reducing a compound of the formula 9

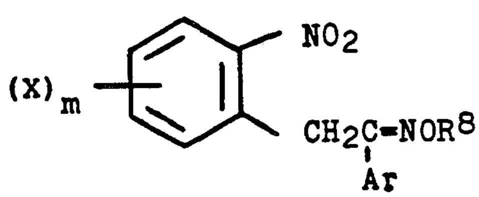

9

wherein Ar, X and m are as defined above, and $R^8$ is a $C_1$-$C_5$-alkanoyl, to afford a compound of the formula II, where $R^3$ is $NH_2$ and $R^1$ and $R^5$ are both hydrogen.

Claims for the following Contracting States: ES,GR

1. A process for the preparation of a compound of the formula I

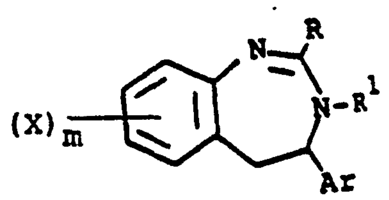

I

wherein Ar is a radical of the following formulae of

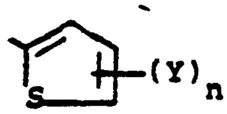
,
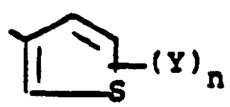
,
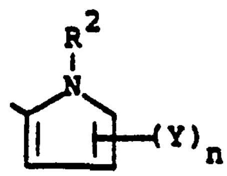
,
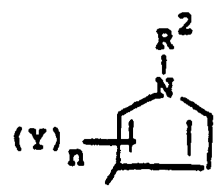

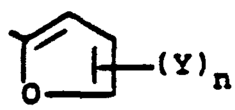
,
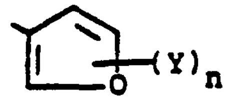
,

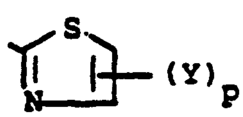
,
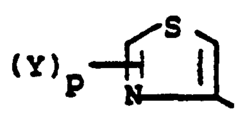
,
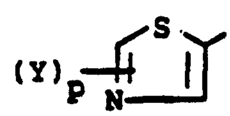
,

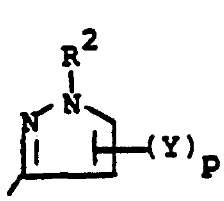
,
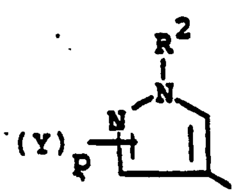
,
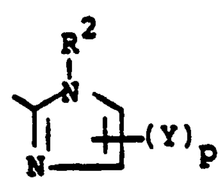
,

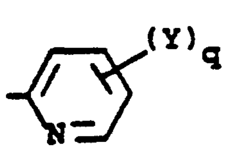
,
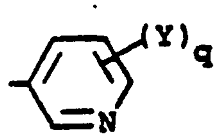
, or
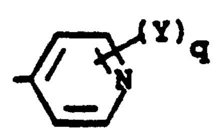

wherein $R^2$ is hydrogen, loweralkyl or loweralkanoyl, Y is halogen, hydroxyl, loweralkyl, loweralkoxy, or trifluoromethyl, n is an integer having a value from 0 to 3 inclusive, p is an integer having a value of 0 or 1, and q is an integer having a value from 0 to 4 inclusive; X is halogen, hydroxyl, nitro, loweralkyl, loweralkoxy, or trifluoromethyl; m is an integer having a value from 0 to 2 inclusive; R is hydrogen, loweralkyl, aryl, aralkyl, cycloalkylloweralkyl, loweralkenyl, or loweralkynyl; and $R^1$ is hydrogen, loweralkyl,

or aralkyl, wherein for each value of m, n, p, or q each X or Y may be the same or different; the optical antipodes, geometrical isomers and pharmaceutically acceptable acid addition salts thereof,
which comprises reacting a compound of the formula 5

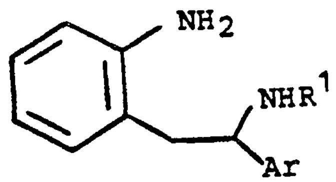

5

wherein $R^1$ and Ar are as defined in claim 3, with a compound of the formula

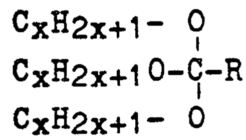

wherein R is as defined in claim 1 and x is an integer having a value of 1 or 2.

2. A process as defined in claim 1 wherein $R^1$ is methyl, m is 0 or 1 and Ar is a radical of the formulae

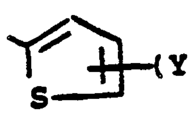
,
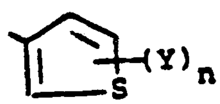
,

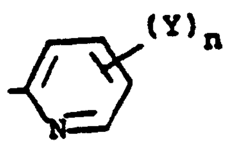
,
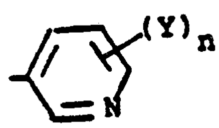
, and
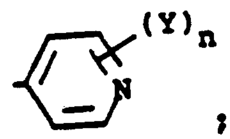
;

wherein Y is loweralkyl and n is an integer having a value of 0 or 1.

3. A process as defined in claim 2 wherein R is loweralkyl.

4. The process as defined in claim 3 wherein 4,5-dihydro-2-ethyl-3-methyl-4-(2-thienyl)-3H-1,3benzodiazepine, or a pharmaceutically acceptable acid addition salt thereof is prepared.

5. The process as defined in claim 3 wherein 4,5-dihydro-2-ethyl-3-methyl-4-(3-methyl-2-thienyl)-3H-1,3-benzodiazepine, or a pharmaceutically acceptable acid addition salt thereof, is prepared.

6. Use of a compound as defined in claim 1 for the preparation of a medicament having antidepressant activity.

7. A process for the preparation of a compound of the formula II

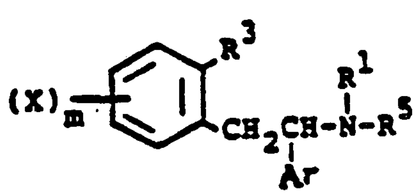

II

wherein Ar is a radical of the formulae

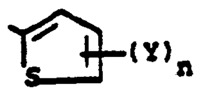, 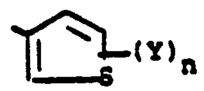, 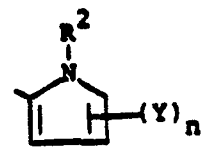, 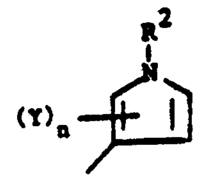

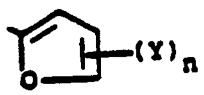, 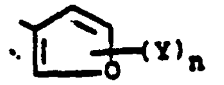,

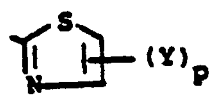, 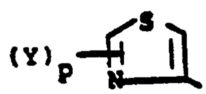, 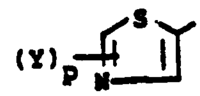,

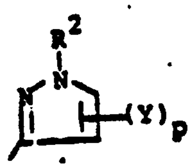, 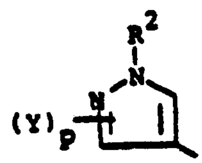, 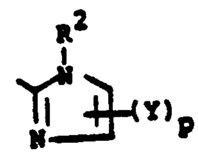,

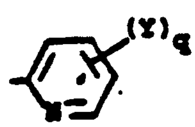, 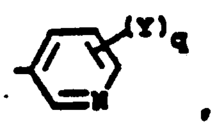, or 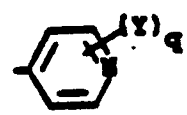

wherein $R^2$ is hydrogen, loweralkyl or loweralkanoyl, Y is halogen, hydroxyl, loweralkyl, loweralkoxy, or trifluoromethyl, n is an integer having a value from 0 to 3 inclusive, p is an integer having a value of 0 or 1, and q is an integer having a value from 0 to 4 inclusive; X is halogen, hydroxyl, nitro, loweralkyl, loweralkoxy, or trifluoromethyl; m is an integer having a value from 0 to 3 inclusive; $R^1$ is hydrogen, loweralkyl, or aralkyl; $R^3$ is hydroxy, loweralkoxy, amino, loweralkylamino, diloweralkylamino or $NR^6C(O)R^4$ wherein $R^4$ and $R^6$ are independently hydrogen or loweralkyl; and $R^5$ is hydrogen, loweralkyl or $C(O)R^7$ where $R^7$ is hydrogen or loweralkyl; wherein for each value of m, n, p, or q each X or Y may be the same or different; the geometrical isomers, optical antipodes and pharmaceutically acceptable acid addition salts thereof, which comprises

a) reacting a compound of the formula 2

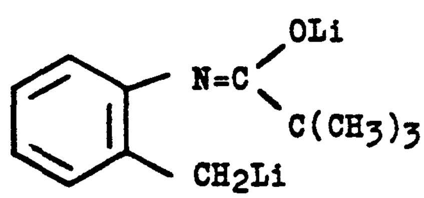

2

wherein X and m are as defined, with a compound of the formula $ArCH=NR^1$ where Ar and $R^1$ are as defined in claim 8 to afford a compound of the formula 4

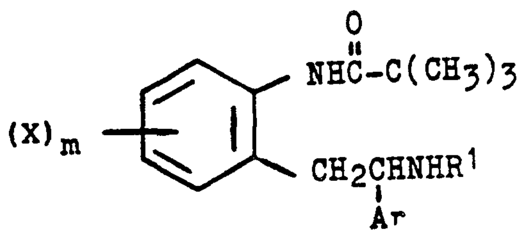

b) hydrolyzing the compound obtained to afford a compound of the formula II, where $R^3$ is amino, $R^1$ and Ar are as defined and $R^5$ is hydrogen,

c) optionally reacting a compound of the formula II, wherein $R^3$ is amino, $R^5$ is hydrogen and $R^1$ and Ar are as defined, with an alcanoic acid anhydride to afford a compound of the formula II, wherein $R^3$ is amino or the group $-NR^6C(O)R^4$, where $R^6$ is hydrogen and $R^4$ is hydrogen or loweralkyl, $R^5$ is the group $-\overset{O}{\overset{\|}{C}}-R^7$ where $R^7$ is hydrogen or loweralkyl, and $R^1$ and Ar are as defined,

d) optionally reducing a compound as obtained in step c) to afford a compound of the formula II, where $R^3$ is the group $-NHR'$ where $R'$ is hydrogen or loweralkyl, $R^5$ is loweralkyl and $R^1$ and Ar are as defined,

e) optionally treating a compound of the formula II wherein $R^3$ is amino, $R^5$ is the group $-\overset{O}{\overset{\|}{C}}-R^7$ where $R^7$ is hydrogen or loweralkyl and $R^1$ and Ar are as defined, with formaldehyde and succinimide followed by reduction of the resultant dioxo-pyrrolidinyl substituted compound of the formula 6c

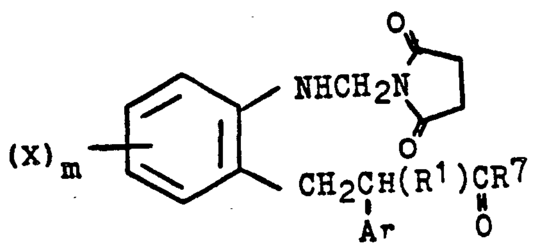

6c

to afford a compound of the formula II where $R^3$ is $-NHCH_3$ $R^5$ is the group $-\overset{O}{\overset{\|}{C}}R^7$, where $R^7$ is hydrogen or loweralkyl, and $R^1$ and Ar are as defined,

f) optionally hydrolyzing the compound as obtained in step c) to afford a compound of the formula II where $R^3$ is $-NHCH_3$, $R^5$ is hydrogen or loweralkyl and $R^1$ and Ar are as defined,

g) optionally diazotizing a compound of the formula II, where $R^3$ is amino, $R^1$ is loweralkyl, $R^5$ is the group $-\overset{O}{\overset{\|}{C}}R^7$ where $R^7$ is hydrogen or loweralkyl and Ar is as defined above, followed by quenching the resultant compound into aqueous copper sulfate solution, to afford a compound of the formula II, where $R^3$ is hydroxy, $R^1$ is loweralkyl, $R^5$ is the group $-\overset{O}{\overset{\|}{C}}R^7$ where $R^7$ is hydrogen or loweralkyl and Ar is as defined above,

h) optionally hydrolyzing a compound as obtained in step g) to afford a compound of the formula II where $R^3$ is hydroxy, $R^1$ is loweralkyl, $R^5$ is hydrogen and Ar is as defined, or

i) reducing a compound of the formula 9

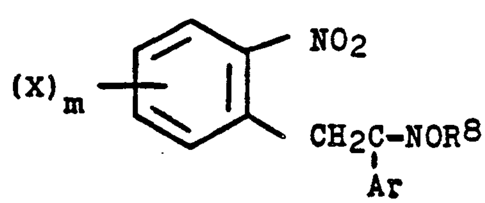

9

wherein Ar, X and m are as defined above, and $R^8$ is a $C_1$-$C_5$-alkanoyl, to afford a compound of the formula II, where $R^3$ is $NH_2$ and $R^1$ and $R^5$ are both hydrogen.

8. A process as defined in claim 7 wherein $R^3$ is amino, m is 0 or 1 and Ar is a radical of the formulae

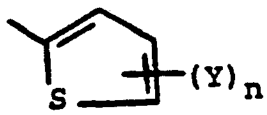

or

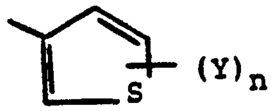

wherein Y is loweralkyl and n is an integer having a value of 0 or 1.

9. The process as defined in claim 9 wherein 2-amino-N-methyl-α-(3-methyl-2-thienyl)-benzeneethanamine or a pharmaceutically acceptable acid addition salt thereof, is prepared.

10. The process as defined in claim 9 wherein 2-amino-5-bromo-N-methyl-α-(3-methyl-2-thienyl)-benzeneethanamine or a pharmaceutically acceptable acid addition salt thereof, is prepared.

11. Use of a compound as defined in claim 7 for the preparation of a medicament having anticonvulsant activity.